# EUROPÄISCHE PATENTSCHRIFT

(11) **EP 0 669 001 B1**
(45) Veröffentlichungstag und Bekanntmachung des Hinweises auf die Patenterteilung: **10.09.1997**
(21) Anmeldenummer: 94900137.4
(22) Anmeldetag: 12.11.1993
(51) Int. Cl.: G01N 33/68, G01N 33/564, C07K 7/04

(54) **BESTIMMUNG VON PEPTIDMOTIVEN AUF MHC-MOLEKÜLEN**
DETERMINATION OF PEPTIDE MOTIFS ON MHC MOLECULES
DETERMINATION DE MOTIFS PEPTIDIQUES SUR DES MOLECULES DU CMH

(30) Priorität: 13.11.1992 DE 4238416
(43) Veröffentlichungstag der Anmeldung: 30.08.1995
(73) Patentinhaber: MAX-PLANCK-GESELLSCHAFT ZUR FÖRDERUNG DER WISSENSCHAFTEN E.V., 14195 Berlin (DE)
(72) Erfinder: RÖTZSCHKE, Olaf, Sommerville, MA 02143 (US); FALK, Kirsten, Sommerville, MA 02143 (US); STEVANOVIC , Stefan, D-68723 Plankstadt (DE); JUNG, Günther, D-72076 Tübingen (DE); RAMMENSEE, Hans-Georg, D-72070 Tübingen (DE)
(74) Vertreter: Weickmann, Heinrich, Dipl.-Ing.
(86) Internationale Anmeldenummer: EP9303175
(87) Internationale Veröffentlichungsnummer: WO9411738

(56) Entgegenhaltungen:
- WO-A-88/05784
- WO-A-92/21033
- PEPTIDES: CHEMISTRY AND BIOLOGY. PROCEEDINGS OF THE 12TH. AMERICAN PEPTIDE SYMPOSIUM. 21. Juni 1991 , CAMBRIDGE , MASSACHUSETTS, USA Seiten 832 - 834 OLAF RÖTZSCHKE ET AL. 'Sequence motifs of peptides eluted from MHC molecules are allele specific'
- NATURE. Bd. 351, Nr. 6324 , 23. Mai 1991 , LONDON GB Seiten 290 - 296 KIRSTEN FALK, OLAF RÖTZSCHKE ET AL. 'Allele-specific motifs revealed by sequencing of self-peptides eluted from MHC molecules'
- EUROPEAN JOURNALOF IMMUNOLOGY Bd. 21, Nr. 11 , November 1991 , WEINHEIM, DE Seiten 2891 - 2894 OLAF RÖTZSCHKE ET AL. 'Exact prediction of natural T cell epitope'
- JOURNAL OF EXPERIMENTAL MEDICINE Bd. 175 , Juni 1992 , NEW YORK, NY, USA Seiten 1799 - 1803 HARALD KROPSHOFER ET AL. 'Self -Peptide Released from Class II HLA-DR1 Exhibits a Hydrophobic Two-Residue Contact Motif'
- JOURNAL OF EXPERIMENTAL MEDICINE Bd. 174 , August 1991 , NEW YORK, NY, USA Seiten 425 - 436 KIRSTEN FALK, OLAF RÖTZSCHKE ET AL. 'Identification of Naturally Processed Viral Nonapeptides Allows Their Quantification in Infected Cells and Suggests an Allele-specific T Cell Eoitope Forecast'
- JOURNAL OF EXPERIMENTAL MEDICINE Bd. 175 , April 1992 , NEW YORK, NY, USA Seiten 961 - 971 R. PAUL JOHNSON ET AL. 'Identification of Overlapping HLA Class I-restricted Cytotoxic T Cell Epitoes in a Conserved Region of the Human Immunodeficiency Virus Type 1 Envelope Glycoprotein: Definition of Minimum Epitopes and Analysis of the Effects of Sequence Variation'
- JOURNAL OF EXPERIMENTAL MEDICINE Bd. 175 , März 1992 , NEW YORK, NY, US Seiten 809 - 820 SARAH E. BUXTON ET AL. 'Anchoring Pockets in Human Histocompatibility Complex Leukocyte Antigen (HLA) Class I Molecules: Analysis of the Conserved B ("45") Pocket of HLA-B27'

## Beschreibung

Die vorliegende Erfindung betrifft ein Verfahren zur Bestimmung von Peptidmotiven bzw. -epitopen auf Molekülen des Major Histocompatibility Complex (MHC) sowie die dadurch bestimmten Peptidmotive und ihre Verwendung zur Herstellung eines diagnostischen oder therapeutischen Mittels.

Die cytotoxischen T-Lymphozyten (CTL) erkennen antigene Peptidepitope in Verbindung mit MHC-kodierten Molekülen. Dieses Phänomen wird als MHC-Restriktion bezeichnet (1-5). Die Kristallographie von menschlichen MHC Klasse I-Molekülen, HLA-2 und Aw68, ergab einen Spalt, der durch die α1- und α2-Domänen der schweren Ketten gebildet wird (3,6). Man nimmt an, daß dieser Spalt die Bindestelle für antigene Peptidepitope ist, da beide Kristalle Strukturen von Peptidgröße enthielten, die nicht mit MHC-Sequenzen kompatibel waren und sich an diesem Spalt befanden (6).

Es wird angenommen, daß diese Peptide von intrazellulären Proteinen stammen und an der Zelloberfläche präsentiert werden, um den cytotoxischen T-Lymphozyten zu erlauben, die Zellen auf abnormale Eigenschaften zu testen. Es wurden bereits MHC-assoziierte Peptide, die T-Zellepitope repräsentieren, aus normalen oder virusinfizierten Zellen extrahiert (2,4,5,7,8). Auf entsprechende Weise können auch Antigene, die durch die MHC Klasse II restringierten T-Zellen erkannt werden, durch künstliche Peptide nachgeahmt werden (9), und MHC-assoziierte antigene Peptide wurden von MHC Klasse II-Molekülen eluiert (10). Aufgrund ihrer Position in der Mitte von trimolekularen Komplexen, die aus T-Zellrezeptor, Peptid und MHC-Molekül bestehen (11), sind die T-Zellepitope ein zentraler Punkt des spezifischen Immunsystems und somit besteht ein großes Bedürfnis nach dem Verständnis der Gesetzmäßigkeiten ihres Auftretens sowie nach einem Bestimmungsverfahren (12-15).

Die erfindungsgemäße Aufgabe wird gelöst durch ein Verfahren zur Bestimmung von allelspezifischen Peptidmotiven auf Molekülen des Major Histocompatibility Complex (MHC) der Klassen I oder II, wobei man
(a) durch Aufschluß von Zellen, die MHC-Moleküle enthalten, einen Zellextrakt erzeugt,
(b) MHC-Moleküle mit den darauf befindlichen Peptidmischungen durch Immunpräzipitation aus dem Zellextrakt abtrennt,
(c) die Peptidmischungen von MHC-Molekülen und sonstigen Proteinbestandteilen abtrennt,
(d) einzelne Peptide oder/und ein Gemisch davon sequenziert, und
(e) aus den erhaltenen Informationen, insbesondere aus der Sequenzierung eines Gemisches, oder aus der Sequenzierung einer Reihe von Einzelpeptiden, das allelspezifische Peptidmotiv ableitet,
welches dadurch gekennzeichnet ist, daß man Peptidmotive auf Molekülen bestimmt, die aus der Gruppe, bestehend aus HLA-A1, HLA-A3, HLA-A11, HLA-A24, HLA-A31, HLA-A33, HLA-B7, HLA-B8, HLA-B*2702, HLA-B*3501, HLA-B*3503, HLA-B37, HLA-B38, HLA-B∗3901, HLA-B*3902, HLA-B*5101, HLA-B*5102, HLA-B*5103, HLA-B∗5201, HLA-B58, HLA-B60, HLA-B61, HLA-B62, HLA-B78, HLA-Cw*0301, HLA-Cw*0401, HLA-Cw*0602, HLA-Cw*0702, HLA-Cw4, HLA-Cw6, HLA-Cw7, HLA-DRB1*0101, DRB1*1201, HLA-DR4w14, HLA-DR17, HLA-DRw52, HLA-DPw2, HLA-DPB1*0401, HLA-DQB1*0301, HLA-DQw1, HLA-DR1, HLA-DR3 und HLA-DR5 ausgewählt sind.

Durch das erfindungsgemäße Verfahren werden Peptidmotive bestimmt, welche die Gesetzmäßigkeiten beinhalten, nach denen MHC-Moleküle Peptide auswählen und präsentieren.

Das erfindungsgemäße Verfahren kann sowohl mit MHC-Molekülen der Klasse I als auch mit MHC-Molekülen der Klasse II durchgeführt werden, wobei MHC-Moleküle der Klasse I bevorzugt sind. Die Peptidmotive HLA-A, HLA-B und HLA-C sind Liganden für MHC-Moleküle der Klasse I. Die Peptidmotive HLA-DR, HLA-DQ und HLA-DP sind Liganden für MHC-Moleküle der Klasse II.

Bei der Immunpräzipitation der MHC-Moleküle durch das erfindungsgemäße Verfahren werden günstigerweise Antikörper verwendet, die für die jeweils gewünschten MHC-Moleküle spezinisch sind. Zur Bestimmung von H-2K^{d}- oder H-2D^{b}-Molekülen werden beispielsweise K^{d}-spezifische Antikörper (25) oder Db-spezifische Antikörper (26) verwendet. Vorzugsweise verwendet man monoklonale Antikörper, es ist jedoch auch die Verwendung eines entsprechend gereinigten polyklonalen Antiserums möglich. Antikörper, die erfindungsgemäß verwendet werden können, können mittels dem Fachmann gut bekannten Standardtechniken de novo hergestellt werden. Beispiele von Antikörpern, die in der Erfindung verwendet werden können, schließen alle Antikörper gegen HLA-Antigene, die in dem "Catalogue of Cell Lines and Hydridomas" des ATCC (American Type Culture Collection, 12301 Parklawn Drive, Rockville, MD 20852) erwähnt sind, mit ein, ohne sich jedoch darauf zu beschränken. Bevorzugte Beispiele (in der ATCC-Nomenklatur) schließen HB82, 117, 166, 54, 122, 164, 95, 120, 116, 118, 94, 152, 178, 56, 115, 157, 119, 59, 105, 165, 144, 180, 103, 110, 109, 151 und 104 mit ein. Alle in dem Katalog erwähnten Antikörper gegen Maus-H-2-Antigene können ebenso in der Erfindung verwendet werden. Besonders bevorzugt erfolgt die Immunpräzipitation durch Festphasen-gebundene Antikörper. Festphasen-gebundene Antikörper lassen sich auf eine dem Fachmann bekannte Weise herstellen, z.B. durch Kopplung des Antikörpers an Bromcyan-aktivierte Sepharose 4B (Pharmacia LKB). Andere Beispiele von Festphasen, an die Antikörper zur erfindungsgemäßen Verwendung gebunden werden können, schließen Agarose, Cellulose, Sephadex, Protein-A-Sepharose und Protein-G-Sepharose mit ein, ohne sich darauf zu beschränken. Das bevorzugte Verfahren der Immunpräzipitation stellt Adsorptions-chromatographie mittels Antikörper, die an aus Cyanogenbromid-aktivierter Sepharose 4B (siehe Beispiel 1) hergestellten Kügelchen gekuppelt sind, dar.

Die Abtrennung der zu bestimmenden Peptidmischungen von MHC-Molekülen und sonstigen Proteinbestandteilen erfolgt günstigerweise durch ein chromatographisches Verfahren, vorzugsweise über Reversed Phase-HPLC. Dabei hat es sich als günstig erwiesen, daß die Abtrennung in einem Trifluoressigsäure/H₂ O-Trifluoressigsäure/Acetonitril-Gradienten erfolgt. Andere Verfahren, die erfindungsgemäß zur Abtrennung von Peptidmischungen von MHC-Molekülen verwendet werden können, schließen Ionenaustausch, Gelfiltration, Elektrofokussierung, High Performance Capillar Elektrophorese (HPCE) und Gelelektrophorese mit ein, sind jedoch nicht darauf beschänkt. Ein anderes Mittel zur Durchführung der Trennung stellt Ultrafiltration dar, wobei eine Membran mit einer Permeabilität von 3000 oder 5000 oder 10000 Da verwendet wird. Bevorzugt wird die Trennung mittels HPLC durchgeführt.

Bei der chromatographischen Auftrennung der Peptidgemische kann man in manchen Fällen eine einzige Peptidspezies isolieren. Somit besteht Schritt (d) des erfindungsgemäßen Verfahrens entweder in der Sequenzierung eines Peptidgemisches, wodurch eine Konsensussequenz für die auf dem jeweiligen MHC-Molekül befindlichen Peptidmotive bestimmt werden kann, oder/und in der Sequenzierung eines definierten Peptids.

Als Ausgangsmaterial für die Bestimmung von Peptidmotiven können normale Zellen, Tumorzellen, als auch durch Viren oder sonstige Erreger infizierte Zellen sowie in vitro kultivierte Zellen des Menschen oder von Tieren verwendet werden. Normale Zellen, die in der Erfindung verwendet werden können, schließen frische Zellen, wie z.B. periphere Blutlymphozyten, Zellen der Milz, der Lunge, des Thymus oder Zellen von einem anderen Gewebe, das MHC-Moleküle exprimiert mit ein, sind jedoch nicht darauf beschränkt. In der Erfindung verwendete Tumorzellinien schließen die Tumorzellen EL4 und P815 mit ein, sind jedoch ebenfalls nicht darauf beschränkt. Virusinfizierte Zellen, die in der Erfindung verwendet werden können, schließen, ohne darauf beschränkt zu sein, JY-Zellen, die durch den Epstein-Barr-Virus transformierte menschliche B-Zellen sind, mit ein. Die durch das erfindungsgemäße Verfahren bestimmten Peptidmotive entsprechen dem folgenden Grundprinzip:
a) Sie weisen eine allelspezifische Peptidlänge von 8, 9, 10 oder 11 Aminosäuren bei MHC-Klasse I-Molekülen sowie von 8 bis 15 Aminosäuren bei MHC-Klasse II Molekülen auf,
b) sie besitzen zwei Ankerpositionen (die Bezeichnung "Ankerposition" wird verwendet, wenn eine Position ein starkes Signal für einen einzigen Aminosäurerest zeigt oder wenn eine Position durch einige wenige Aminosäurereste mit sehr nahe verwandten Seitenketten besetzt wird), wovon sich eine Ankerposition immer am C-terminalen Ende befindet und häufig aliphatisch ist, und
c) die Peptide werden natürlicherweise auf MHC-Molekülen von normalen, virusinfizierten, anderweitig infizierten oder mit Genen transfizierten oder mit Antigen beladenen Zellen präsentiert.

Die Sequenzierung der Selbstpeptidgemische aus den MHC-Klasse I-Molekülen H2K^{d}, H2K^{b}, H2D^{b} und HLA-A2 zeigt ein jeweils unterschiedliches allelspezifisches Peptidmotiv, das von jedem der Klasse I-Moleküle präsentiert wird. Die von K^{d}, D^{b} und A2 präsentierten Peptide sind Nonamere, während die K^{b}-präsentierten Peptide Octamere sind, wobei die korrespondierenden Peptidmotive zwei Ankerpositionen enthalten, die durch einen einzigen Aminosäurerest oder durch einen aus einer geringen Anzahl von Aminosäureresten mit nahe verwandten Seitenketten besetzt sind. Diese Ankerpositionen befinden sich bei den unterschiedlichen Motiven nicht an derselben Stelle, sie können etwa an Position 5 und 9 (D^{b}) oder 2 und 8 (K^{d}, A2) oder 5 und 8 (K^{b}) sein. Die C-terminalen Ankerreste aller Motive sind hydrophobe Aminosäuren. Die nicht an Ankerpositionen befindlichen Aminosäurereste können ziemlich variabel sein, einige jedoch werden vorzugsweise durch bestimmte Aminosäuren besetzt, beispielsweise findet man häufig Pro an Position 4 des K^{d}-Motivs, Tyr an Position 3 des K^{b}-Motivs und hydrophobe Reste herrschen an den Positionen 3 des D^{b}-Motivs und 6 des A2 Motivs vor. Für H-2L^{d} war ein Ankerrest Prolin an Position 2.

Die durch das erfindungsgemäße Verfahren gewonnenen Ergebnisse entsprechen sehr gut der Struktur des kristallographisch gefundenen Spalts bei MHC-Klasse I-Molekülen (3,6). Unterschiedliche MHC-Klasse I-Allele unterscheiden sich an diesem Spalt durch das Vorhandensein unterschiedlicher Taschen, was vermutlich darauf zurückzuführen ist, daß die Taschen jeweils unterschiedliche Aminosäuren aufnehmen können. Daher stellen die allelspezifischen Taschen in den MHC-Kristallen und die Seitenketten der allelspezifischen Ankerreste vermutlich komplementäre Strukturen dar.

Ein weiterer Gegenstand der vorliegenden Erfindung ist die Verwendung der erfindungsgemäßen Peptidmotive bei einem Verfahren zur Herstellung eines diagnostischen oder therapeutischen Mittels. Ein mögliches Anwendungsgebiet der Peptidmotive ist der diagnostische Nachweis von MHC-Molekülen. Da die MHC-Moleküle durch ihre individuelle spezifische Bindung von Peptiden charakterisiert sind, kann ein Bindungsnachweis über Peptide einer Markierungsgruppe erfolgen, wobei als Markierungsgruppe beispielsweise eine Biotin- oder eine Fluoreszenzgruppe an das Peptid gekoppelt wird. Andere dem Fachmann bekannte Markierungen können ebenso in der Erfindung verwendet werden. Diese Markierungen schließen, ohne sich darauf zu beschränken, radioaktive Markierungen wie z.B. an Thyrosinreste von Peptiden gebundenes ¹³¹ I oder ¹²⁵ I, oder ³ H oder ¹⁴ C (beide während deren Synthese in die Peptide eingebaut) mit ein. Bindung der Markierungen an die Peptide kann nach dem Fachmann gut bekannten Verfahren erreicht werden. Die Markierung erfolgt vorzugsweise an Nicht-Ankerpositionen. Die auf solche Weise gefundenen Korrelationen zwischen dem Auftreten von Autoimmunkrankheiten und der Expression von MHC-Molekülen mit krankheitsspezifischen Peptidmotiven können diagnostisch verwertet werden. Beispiele von in vitro diagnostischen Verwendungen der erfindungsgemäßen Peptidsequenzen schließen, ohne sich darauf zu beschränken, Messung der Bindungsspezifität von MHC-Molekülen, Korrelierung der Bindungsspezifität von MHC-Molekülen mit Krankheiten, und Bestimmung der Sequenz von T-Zellepitopen unbekannten Ursprungs durch Inkubieren geeigneter Zellen, die die interessierenden MHC-Moleküle exprimieren mit HPLC-Fraktionen einer Peptid-Bank (Mischung von Peptiden, die in das untersuchte Motiv passen) und Bestimmung der durch die T-Zelle erkannten Peptide, gefolgt von chromatographischem Vergleich des natürlichen T-Zellepitops mit dem als T-Zellepitop erkannten synthetischen Peptid (Nature 348: 252-254 (1990)) mit ein.

Ein weiterer Gegenstand der Erfindung ist die Verwendung der erfindungsgemäßen Peptidmotive bei einem Verfahren zur Herstellung eines Arzneimittels zur Therapie von Störungen des Immunsystems oder von Tumorerkrankungen. Insbesondere können die erfindungsgemäßen Peptidmotive für die Intervention bei Autoimmunkrankheiten (Prophylaxe und Therapie), beispielsweise durch Blockierung bestimmter MHC-Moleküle sowie durch die Induktion peptidspezifischer Nicht-Reaktivität von T-Zellen, verwendet werden. Weiterhin ist eine Intervention bei Transplantatabstoßungen und Graft-versus-Host-Reaktionen auf analoge Weise möglich. Ferner können die erfindungsgemäßen Peptide für die Induktion oder die Verstärkung bzw. Vermehrung von gegen Tumorzellen gerichteten T-Zellen in vitro und in vivo eingesetzt werden, insbesondere für die Vakzinierung gegen Tumorerkrankungen und für die Therapie bestehender Tumorerkrankungen, wobei insbesondere der sogenannte Graftversus-Leukämia-Effekt (Sullivan et al., N.Engl.J.Med. 320: 828-834) ausgenutzt werden kann. Die erfindungsgemäßen Peptide können ebenso dazu verwendet werden, T-Zellantworten gegen infektiöse oder maligne Krankheiten zu verstärken, indem MHC-bindende Peptide, die spezifisch für das infektiöse Mittel oder für Tumore sind, in vivo eingesetzt werden. Alternativ können T-Zellen aus Patienten gewonnen werden, ihre Anzahl in vitro durch Verwendung von Peptiden und geeigneten Wachstumsbedingungen, einschließend Cytokine, wie z.B. Interleukin 2, Interleukin 4 oder Interleukin 6 vermehrt und anschließend in den Patienten zurückgeführt werden. Die erfindungsgemäßen Peptide können weiterhin dazu verwendet werden, alle Tumore, die durch T-Zellen angreifbare Antigene exprimieren, einschließlich, ohne darauf zu beschränken, Melanome, Brustkrebs, Tumore viralen Ursprungs, wie z.B. Burkittslymphom und solche Tumore, die durch menschlichen Papillomavirus wie zervikales Karzinom und andere anogenitale Tumore zu behandeln. Peptide, die von T-Zellrezeptor-Molekülen oder Antikörpermolekülen abstammen, können auch für die gezielte Manipulation immunregulatorischer Mechanismen eingesetzt werden, insbesondere für die Bekämpfung von Autoimmunkrankheiten und Transplantatabstoßungen, sowie Graft-versus-Host-Reaktionen. In vivo-Verwendungen der erfindungsgemäßen Proteine zur Prävention schließen ihre Verwendung, ohne darauf beschränkt zu sein, als Peptidvakzine gegen infektiöse oder maligne Krankheiten und Verwendung der in dieser Erfindung gesammelten Information bezüglich geeigneter T-Zellepitope zu ihrem Einbau in alle anderen Arten von Impfstoffen einschließlich rekombinante Impfstoffe (einschließlich Viruse wie Vaccinia oder Bakterien wie Salmonella oder Mycobacteria) und Proteine, die durch Verwendung von rekombinanten Bakterien (z.B. E.coli) oder anderen Zellen, einschließlich Hefe-, Insekten-, Maus- oder menschlichen Zellen hergestellt wurden, mit ein.

Die Dosierung oder Konzentrationen der erfindungsgemäße Peptide können durch den Fachmann routinemäßig bestimmt werden. Diese können in vivo in einem Bereich von 10 µg bis 1 g erwartet werden. In vitro-Konzentrationen können in einem Bereich von 1 Femtomol bis 1 Micromol erwartet werden. Die Verabreichung in vivo schließt, ohne sich darauf zu beschränken, einen subkutanen, intramuskulären, intravenösen, intradermalen und oralen Weg mit ein.

Vorzugsweise ist bei der therapeutischen Verwendung ein Peptid, das einem erfindungsgemäßen Peptidmotiv entspricht, N- oder/und C-terminal mit lipophilen bzw. amphiphilen Gruppen, insbesondere lipophilen Peptid-Helices kovalent verknüpft. Ein Beispiel für eine derartige Gruppe ist Tripalmitoyl-S-glycerylcysteinyl-serylserin.

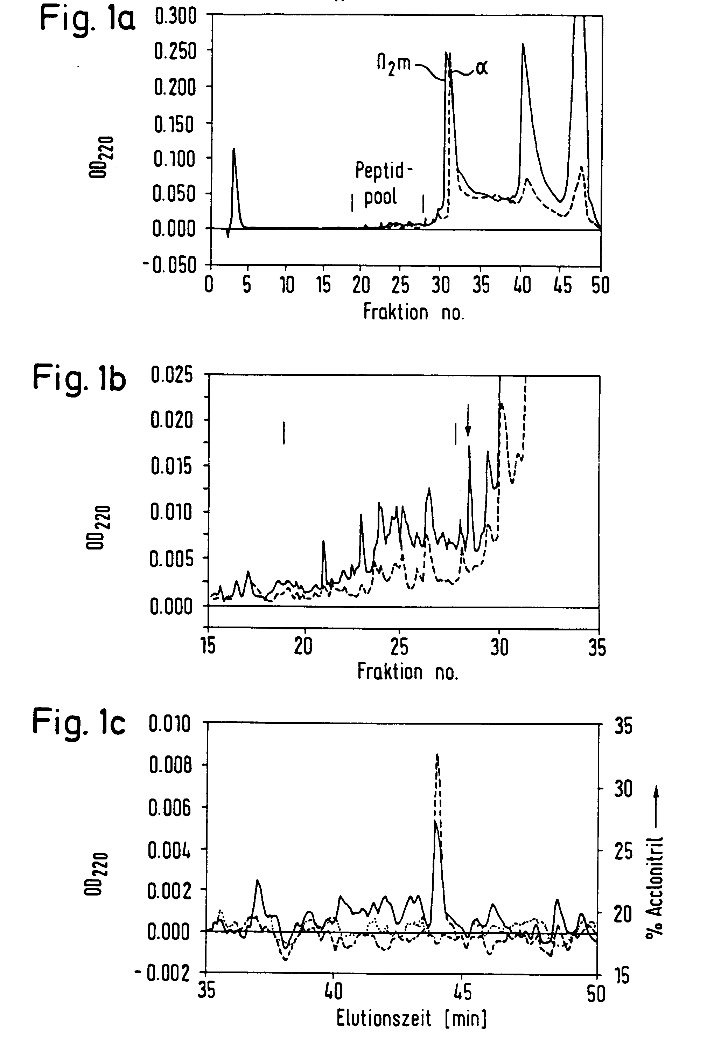

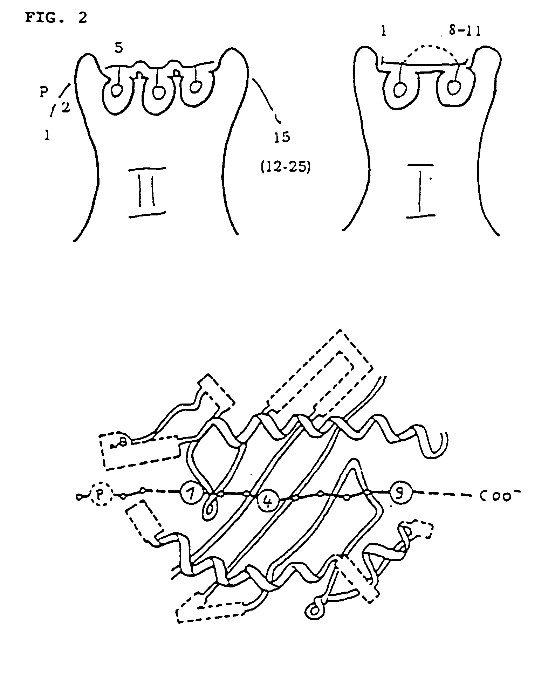

Die Erfindung soll weiter durch die folgenden Beispiele in Verbindung mit den Figuren 1 und 2 veranschaulicht werden.

Es zeigen
- Fig. 1a: ein HPLC-Profil von Material, das mit Anti-K^{d}-Antikörpern aus P815 Lyssat abgetrennt wurde,
- Fig. 1b: einen vergrößerten Ausschnitt des Chromatogramms aus 1a (Fraktionen 15 - 35),
- Fig. 1c: eine Rechromatographie des in 1b mit einem Pfeil gekennzeichneten Selbstpeptids,
- Fig. 2: MHC-Moleküle und ihre Liganden.

### Beispiel 1

10 bis 20x10⁹ P815-Tumorzellen (H-2K^{d}) wurden pelletiert und 30 Minuten mit 250 ml 0,5 % Nonidet P40 in Phosphat-gepufferter Salzlösung (PBS) mit 0,1 mmol/l Phenylmethylsulfonylfluorid (PMSF) bei 4°C gerührt. Der Überstand wurde 5 Minuten bei 250 g und 30 Minuten bei 150.000 g und 4°C) zentrifugiert und dann durch eine adsorptionschromatographische Anordnung geleitet. Die adsorptionschromatographische Anordnung bestand aus drei Säulen mit jeweils einem Bettvolumen von etwa 1 ml. Das Säulenmaterial bestand aus Antikörper-gekoppelten bzw. Glycin-gekoppelten Kügelchen, die aus Bromcyan-aktivierter Sepharose 4B (Pharmacia LKB) gemäß dem Protokoll des Herstellers hergestellt wurden. Als Antikörper wurden jeweils 5 mg von K^{d}-spezifischem Antikörper 20-8-4S (IgG 2a, kappa; 25) oder D^{b}-spezifische Antikörper B22-249 (IgG 2a, kappa; 26) an 1 ml der Kügelchen gekoppelt. Der Überstand des Zellextrakts wurde zunächst durch eine Säule mit Glycin-gekoppelten Kügelchen, dann durch eine entsprechende Säule mit Anti-K^{d}-Kügelchen und dann für eine Scheinpräzipitation über Anti-D^{b}-Kügelchen geleitet.

Die Kügelchen wurden aus allen drei Säulen entfernt und mit 0,1 % Trifluoressigsäure für 15 Minuten verwirbelt (7). Die Überstände wurden durch Vakuumzentrifugation getrocknet und durch Reverse Phase HPLC unter Verwendung einer Superpac Pep S Säule (C2/C18; 5 µm Teilchen, 4,0 x 250 mm, Pharmacia LKB) und einer Pharmacia LKB-Apparatur abgetrennt (4). Elutionsmittel: Lösung A 0,1 % Trifluoressigsäure in H₂O (v/v), Lösung B 0,1 % Trifluoressigsäure in Acetonitril.

Für die in Figur la und b gezeigten chromatographischen Trennungen wurde der folgende Gradient verwendet:
0 bis 5 Minuten, 100 % A
5 bis 40 Minuten linearer Anstieg auf 60 % B,
40 bis 45 Minuten 60 % B,
45 bis 50 Minuten Abnahme auf 0 % B,
Flußrate: 1 ml/Minute, Fraktionsgröße: 1 ml.
Die einzelnen Fraktionen wurden gesammelt und durch Vakuumzentrifugation getrocknet.

Figur 1 zeigt die HPLC-Auftrennung von immunpräzipitierten und Trifluoressigsäure-behandelten K^{d}-Molekülen. Figur la zeigt ein HPLC-Profil von TFA-behandeltem Material, das aus P815-Lysat mit Anti-K^{d} (durchgehende Linie) bzw. mit Anti-D^{b} (gestrichelte Linie) präzipitiert wurde. Zwischen den Fraktionen 20 und 28 wird heterogenes Material in geringen Mengen eluiert, bei dem es sich um die gesuchten allelspezifischen Peptidgemische handelt.

Die Fraktionen 20 bis 28 wurden sowohl aus dem K^{d}-Ansatz als auch von dem Scheinpräzipitat gesammelt. Beide Ansätze wurden unter Verwendung der Edman-Abbaumethode automatisch sequenziert (Edman et al., Eur.J.Biochem. 1: 80-91 (1967)). Der Edman-Abbau wurde in einem Protein Sequencer 477A, ausgestattet mit einem on-line PTH-Aminosäure Analysator 120A (Applied Biosystems, Foster City, CA, 94404, USA) durchgeführt. Glasfaserfilter wurden mit 1 mg BioPrene Plus beschichtet und nicht präzyklisiert. Die Sequenzierung wurde unter Verwendung der Standardprogramme BEGIN-1 und NORMAL-1 (Applied Biosystems) durchgeführt. Cystein wurde nicht modifiziert und konnte deshalb auch nicht nachgewiesen werden.

Das Edman-Verfahren beinhaltet eine sequenzielle Derivatisierung und Aminosäurenentfernung vom N-Terminus, von denen jede chromatographisch identifiziert wird. Da es ungewöhnlich ist, komplexe Gemische von Peptiden zu sequenzieren, werden die direkt aus dem Sequenziergerät gewonnenen Daten präsentiert. Tabelle la und b zeigen die Ergebnisse aus zwei Sequenzierungsversuchen für K^{d}-eluierte Peptide. Tabelle 1c zeigt das Sequenzierungsergebnis einer Scheinelution mit Db-spezifischen Antikörpern auf P815-Lysaten. Die K^{d}-eluierten Peptide haben ein klares Aminosäuremuster für jede Position von 1 bis 9, während das scheineluierte Material durchgehend ein gleichförmiges Aminosäuremuster mit einer Abnahme der absoluten Menge jedes Rests bei jedem Zyklus zeigt. Bei den Kd-eluierten Peptiden wurden nur die Reste, die mehr als 50 % Anstieg in der absoluten Menge im Vergleich mit dem vorherigen oder dem vorvorherigen Zyklus zeigten, als signifikant erachtet und unterstrichen. Die erste Position ist schwierig zu beurteilen, da es keinen vorherigen Zyklus gibt und überdies alle im HPLC-Pool vorhandenen freien Aminosäuren an dieser Position nachgewiesen werden. Für die zweite Position ist der einzige Rest, dessen Häufigkeit im Vergleich zum vorherigen Zyklus klar erhöht ist, Tyrosin (z.B. Tabelle la 60,9 pmol auf 875,6 pmol). Der einzige andere Rest, der einen (geringen) Anstieg zeigt, ist Phenylalanin, das eine zu Tyr ähnliche Seitenkette aufweist. Dies bestätigt die Annahme, die aus einem Vergleich des natürlichen K^{d}-restringierten Influenza-Epitops (mit der Sequenz TYQRTRALV) mit anderen K^{d}-restringierten Peptiden im Hinblick auf den Tyrosin-Rest an Position 2 resultiert. Dagegen gibt es keinen definierten Aminosäurerest, der für die folgenden Positionen 3 bis 8 charakteristisch ist. Es werden bis zu 14 unterschiedliche Reste in den einzelnen Positionen gefunden. An Position 9 werden Ile und Leu gefunden. Es gibt keinen Signalanstieg an Position 10, was darauf hindeutet, daß die meisten K^{d}-gebundenen Selbstpeptide nicht länger als 9 Reste sind. Das natürliche K^{d}-restringierte Influenza-Peptid ist somit ein Nonapeptid (4). Das Konsensussequenzmuster, das aus diesen Ergebnissen hervorgeht, ist in Tabelle 1c gezeigt. Am meisten auffallend sind Tyr an Position 2 und Ile oder Leu an 9, während an allen anderen Positionen eine größere Anzahl an Resten gefunden wird. Ein Vergleich dieses Motivs mit Peptidsequenzen, die K^{d}-restringierte Epitope enthalten, zeigt, daß die meisten gut zu dem K^{d}-restringierten Konsensusmonomer-Motiv passen (Tabelle 1d).

Der durch einen Pfeil in Fraktion 29 von Figur 1b markierte Peak und die korrespondierende Fraktion der Scheinpräzipitation wurden unter höherer Auflösung erneut chromatographiert, wobei das Fraktionsvolumen 0,5 ml betrug (Fig. 1c). Der scharfe spezifische Peak stellte ein Peptid mit der Aminosäuresequenz SYFPEITHI dar, das durch direkte Sequenzierung bestimmt wurde. Die Identität dieses natürlichen Zellpeptids mit synthetischem SYFPEITHI-Peptid wurde durch Coelution auf HPLC bestätigt (Fig. 1c). Die Sequenz paßt zu dem Konsensusmotiv aus dem Pool der Fraktionen 20 bis 28 (Fig. la,b), wodurch das Vorhandensein eines spezifischen Kd-restringierten Peptidmotivs (Tabelle 1d) bestätigt wird.

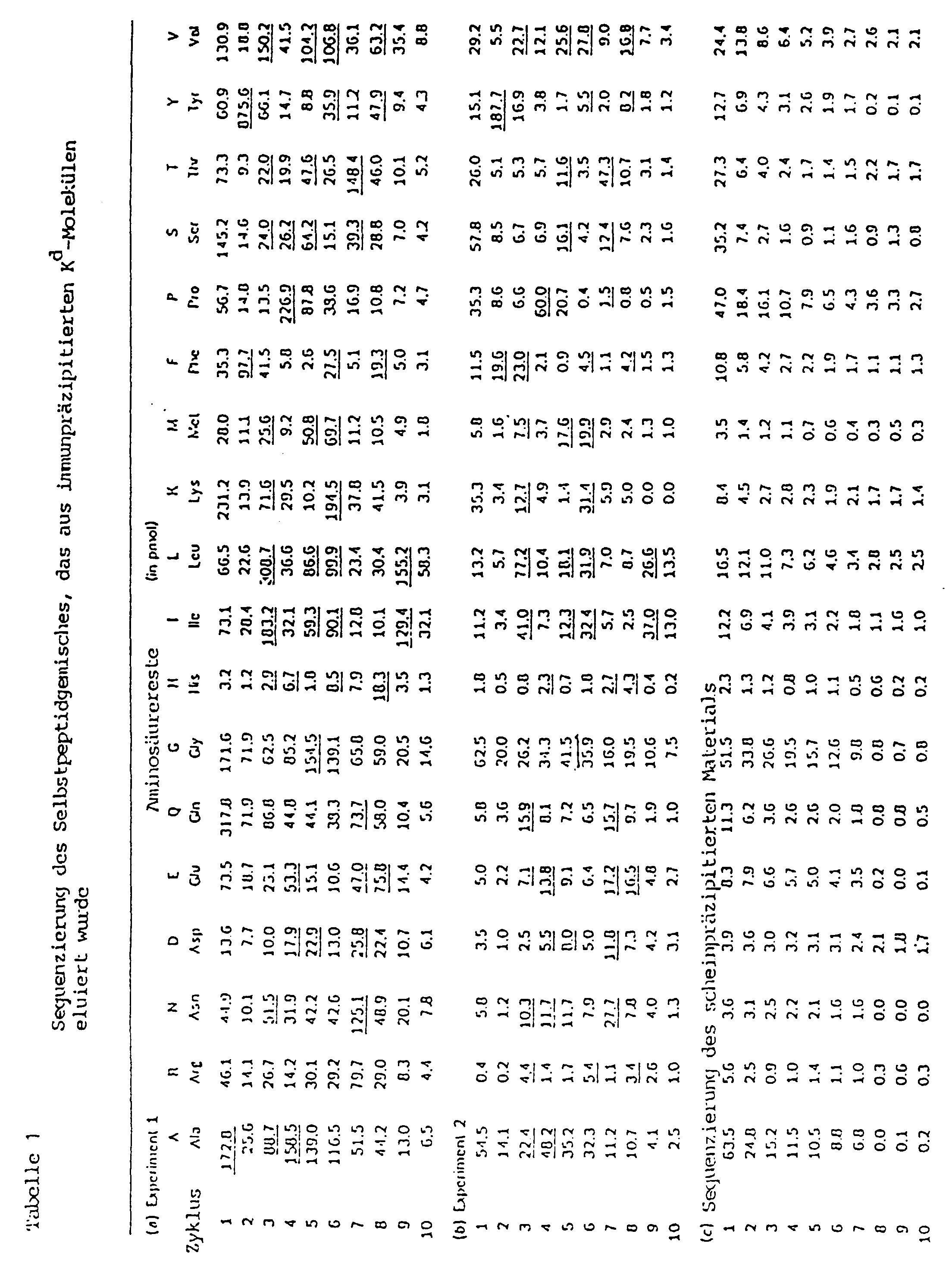

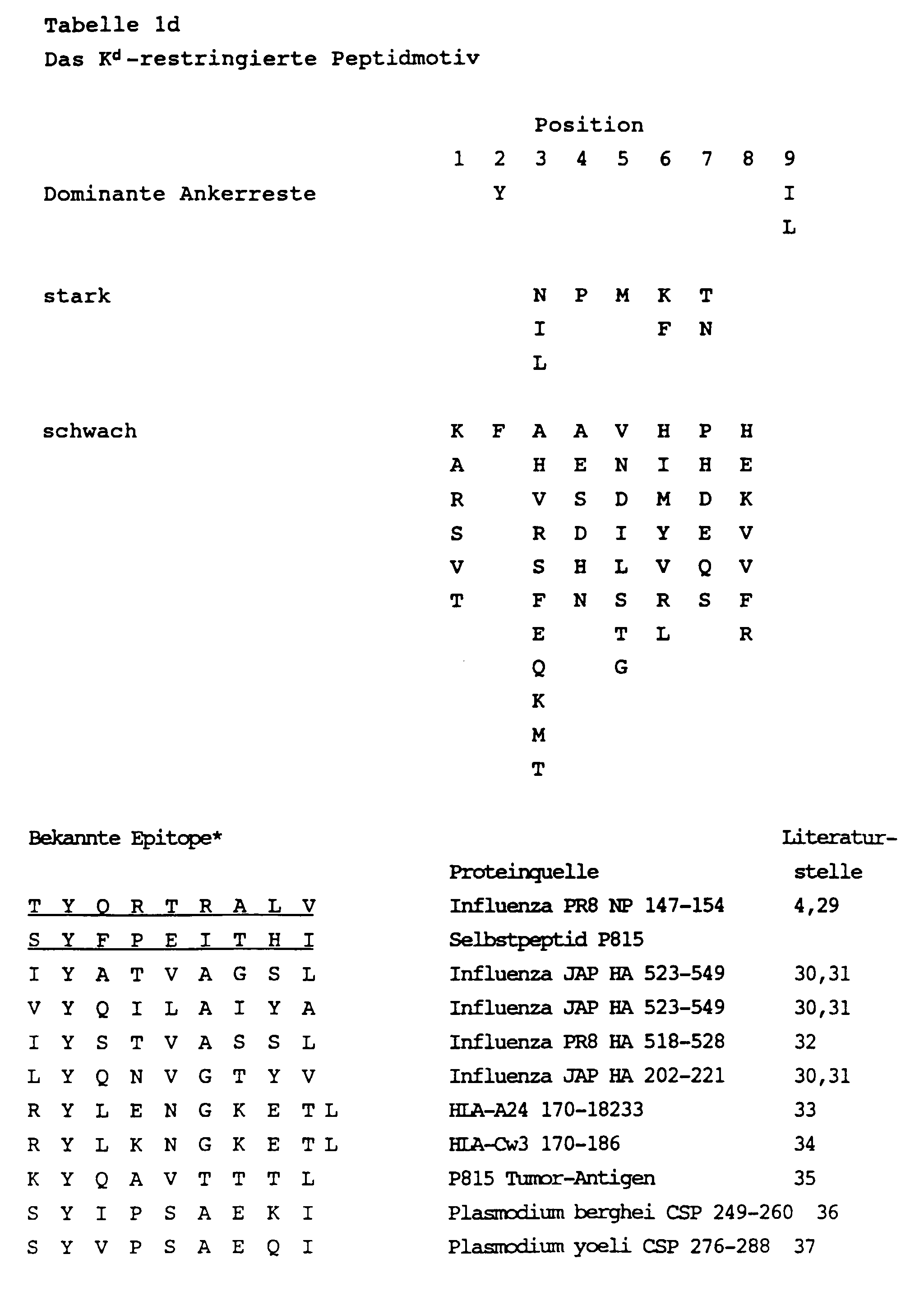

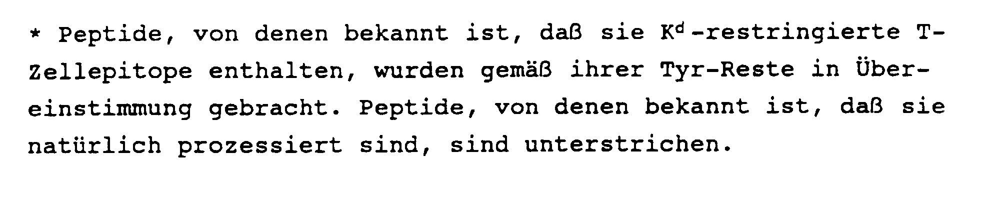

### Beispiel 2

### Elution von Peptiden aus K^{b}- und D^{b}-Molekülen

Detergenz-Lysate aus EL4-Tumorzellen (H-2^{b}) wurden mit K^{b}-spezifischen und D^{b}-spezifischen Antikörpern, wie in Beispiel 1 beschrieben, immunpräzipitiert. Als D^{b}-Antikörper wurde B22-249 (siehe Beispiel 1) und als K^{b}-Antikörper wurde K9-178 (IgG 2a, K, 27) verwendet. Die von MHC-Molekülen dissoziierten Peptide wurden durch Reverse Phase HPLC aufgetrennt. Sowohl K^{b}- als auch D^{b}-Material wurde mit Profilen eluiert, die in etwa dem K^{d}-Material aus Beispiel 1 entsprachen, wobei jedoch in dem heterogenen Material, das zwischen Fraktionen 20 und 28 eluierte, gewisse Unterschiede auftraten.

### D^{b}-restringiertes Peptidmotiv

Die vereinigten Fraktionen 20 bis 28 aus dem D^{b}-Ansatz wurden sequenziert (Tabelle 2a,b). Die Positionen 2 bis 4 enthielten mehrere Reste. Dagegen gab Zyklus 5 ein starkes Signal für Asn. Der vorherrschende Rest an Position 5 der D^{b}-eluierten Selbstpeptide ist somit Asn. Das schwache Signal für Asp wird durch Hydrolyse von Asn zu Asp unter den Sequenzierungsbedingungen verursacht. Die Positionen 6 bis 8 enthielten 5 bis 14 unterschiedliche nachweisbare Reste. Position 9 enthielt ein starkes Signal für Met, ein mittleres für Ile und ein schwaches für Leu (alle hydrophob). (Die Bedeutung von Met oder Ile in einem D^{b}-restringierten Epitop wurde bereits berichtet, siehe 17). An Position 10 war kein Signal, was darauf hindeutet, daß D^{b}-präsentierte Selbstpeptide Nonapeptide sind. Das durch diese Ergebnisse ermittelte Konsensusmotiv ist in Tabelle 2c gezeigt. Ein Vergleich dieses Motivs mit dem natürlichen D^{b}-restringierten Peptid und mit anderen Peptiden, die D^{b}-restringierte Epitope enthalten, zeigt, daß Asn an Position 5 ein unveränderlicher Ankerrest des D^{b}-restringierten Peptidmotivs sein kann. Die anderen Reste der D^{b}-restringierten Epitope unterscheiden sich erheblich, mit Ausnahme von Position 9 (mit Met, Ile oder Leu), die wie eine zweite Ankerposition aussieht.

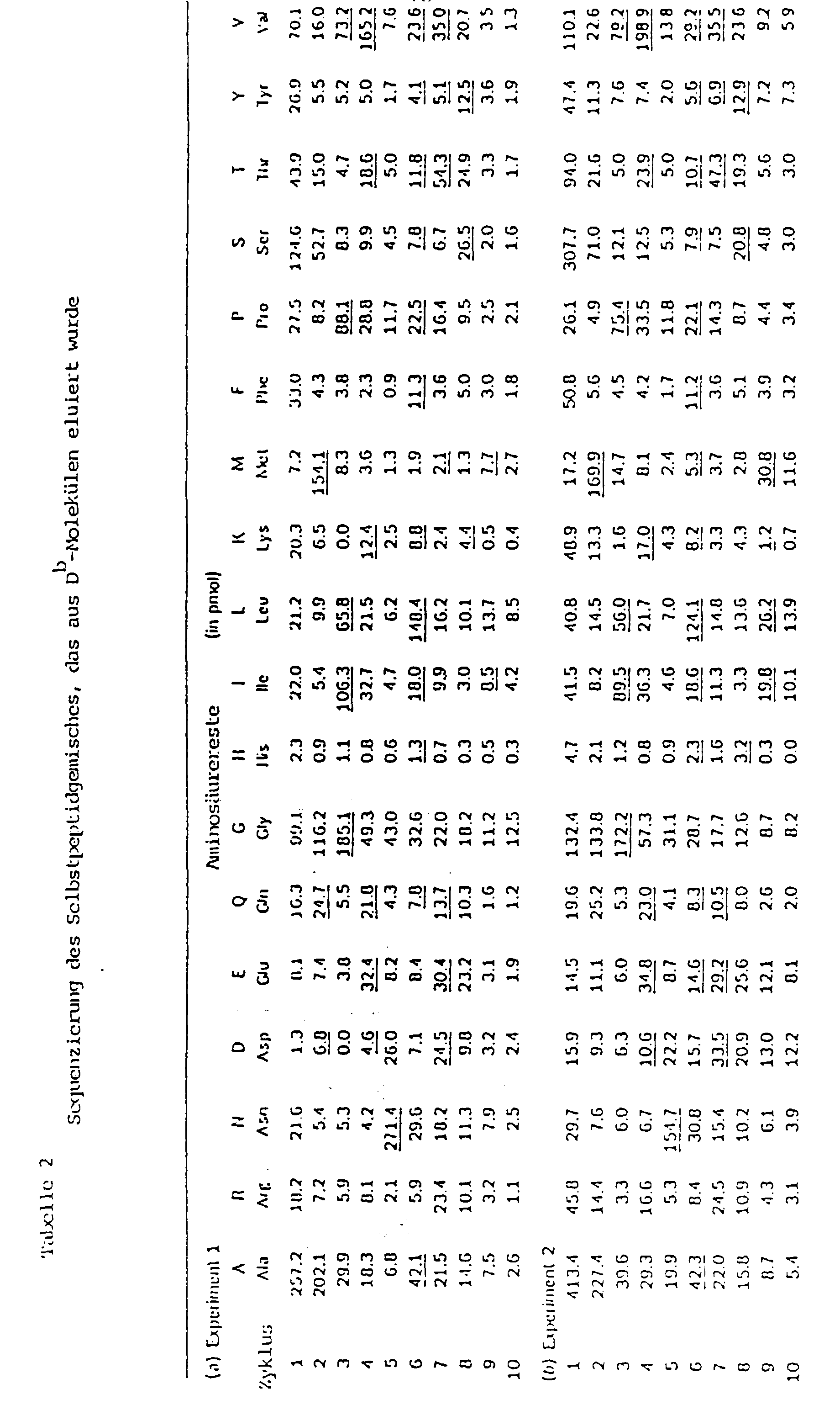

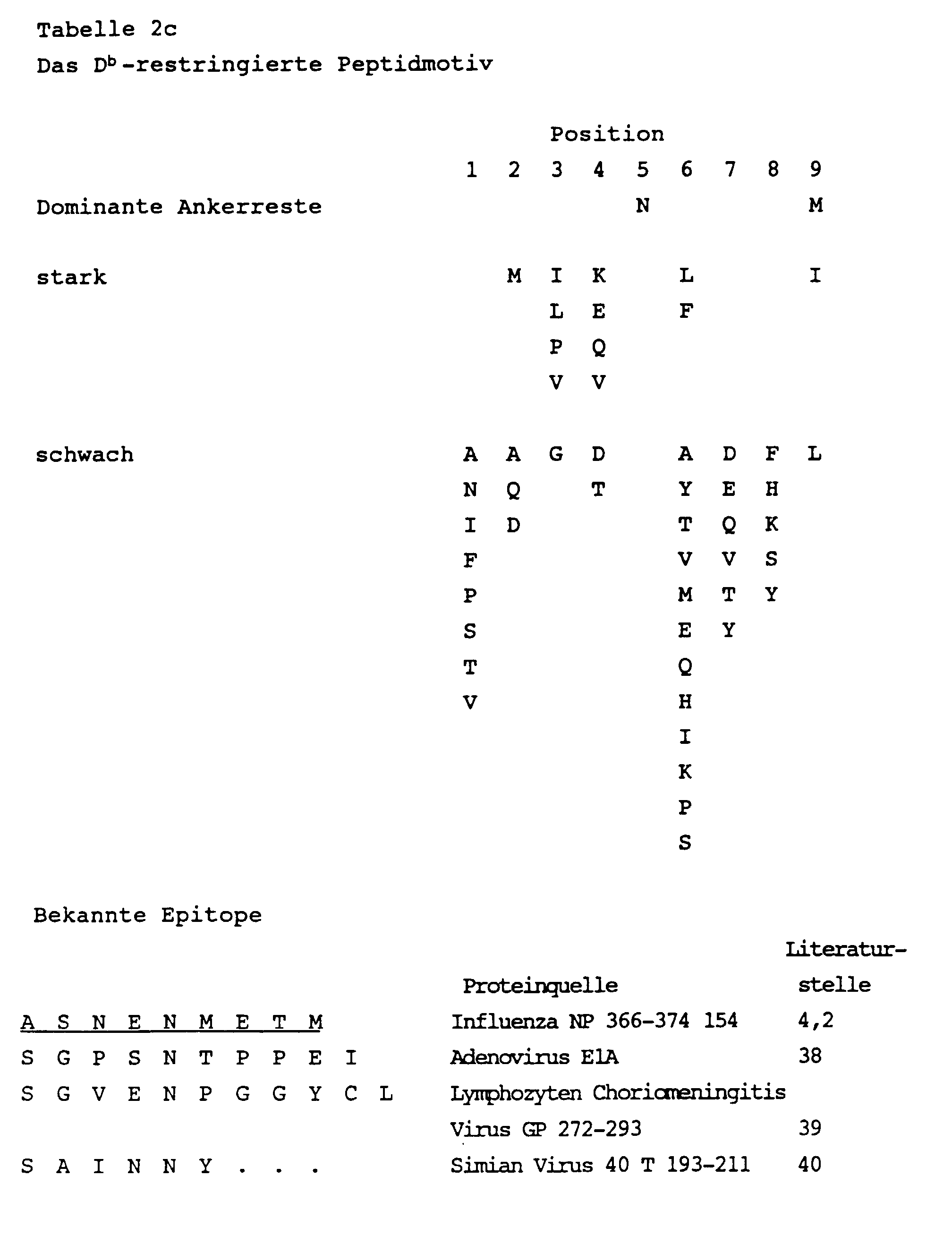

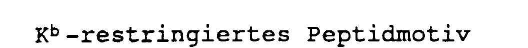

Die vereinigten Fraktionen 20 bis 28 aus dem K^{b}-Ansatz wurden sequenziert (Tabelle 3a,b). Position 3 enthielt ein starkes Signal für Tyr und ein schwaches für Pro. Position 4 zeigte schwache Signale für 5 Reste. Starke Signale für Phe und für Tyr machen diese beiden Reste an Position 5 vorherrschend. Die nächsten beiden Positionen enthielten 5 bzw. 3 Signale. Position 8 zeigte ein starkes Signal für Leu, ein mittleres für Met und schwächere für Ile und Val. Position 9 zeigte keinen Anstieg für irgendeinen Rest, was mit der Länge des bekannten K^{b}-restringierten natürlichen Peptids, das ein Octamer ist (5), übereinstimmt. Eine Analyse des K^{b}-restringierten Konsensusmotivs und Vergleich mit Epitopen zeigt zwei Ankerpositionen: Tyr oder Phe (beide mit ähnlichen aromatischen Seitenketten) an Position 5 und Leu, Met, Ile oder Val (alle mit ähnlichen hydrophoben Seitenketten) an Position 8.

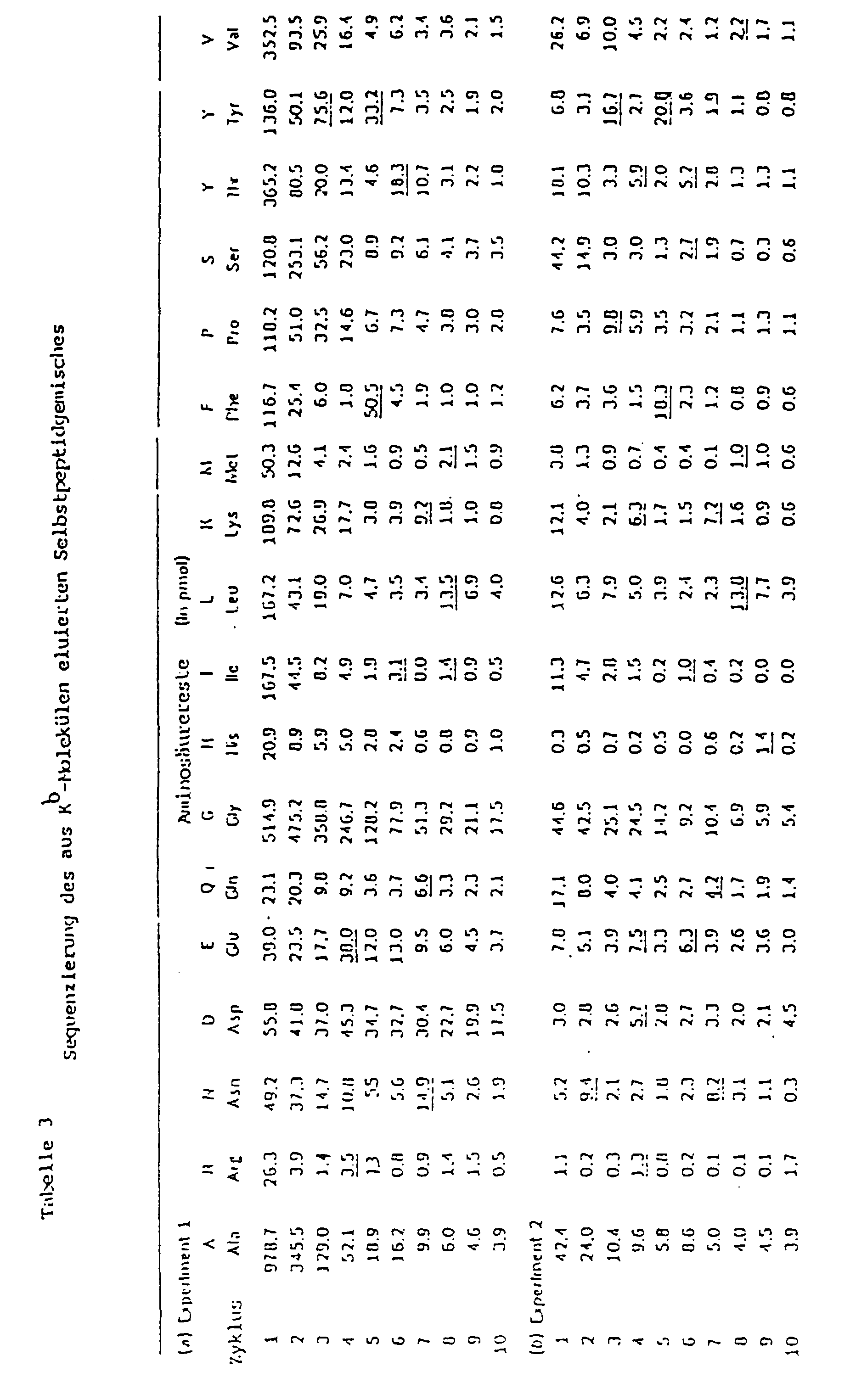

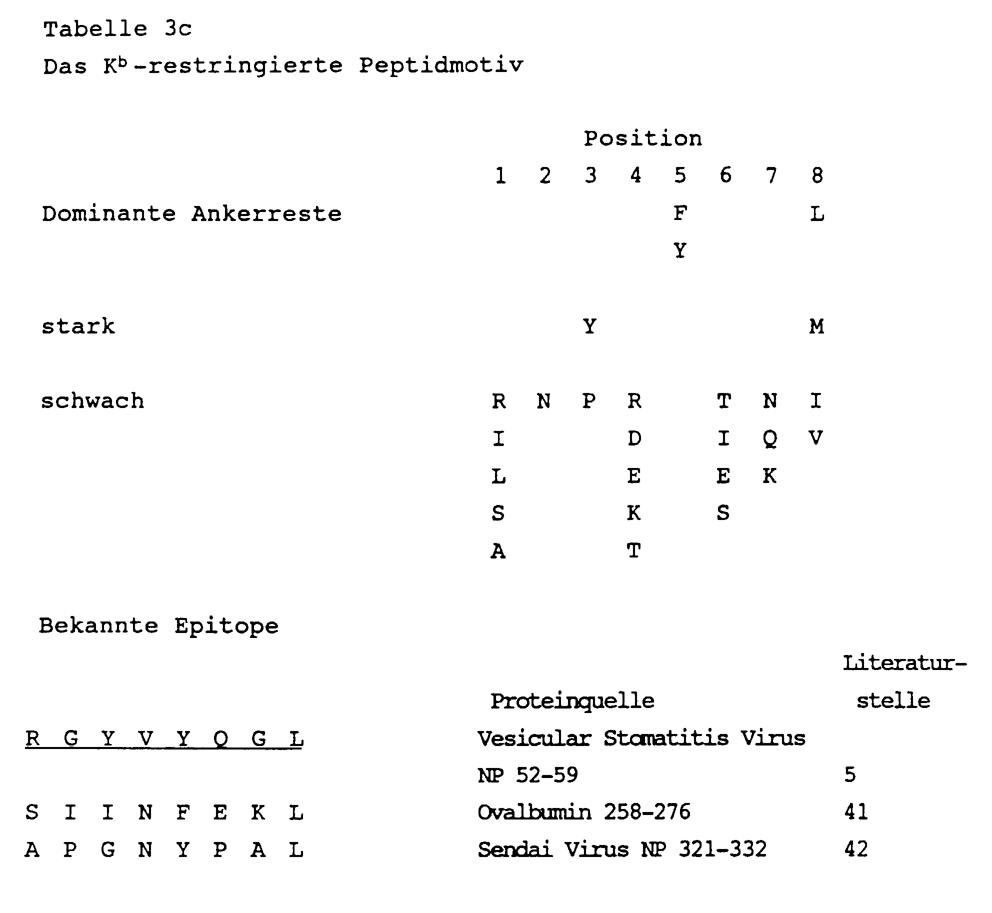

### Beispiel 3

### HLA-A2.1-restringiertes Peptidmotiv

Ein Detergenz-Lysat von menschlichen JY-Zellen mit dem HLA-A2.1-MHC-Molekül (45) wurde mit A2-spezifischen Antikörpern (BB7.2, IgG2b, Literaturstelle 28) immunpräzipitiert. Die von A2-Molekülen dissoziierten Peptide wurden durch HPLC aufgetrennt. Es wurden die Fraktionen 20 bis 28 vereinigt und wie zuvor beschrieben sequenziert (Tabelle 4). Die zweite Position enthielt ein starkes Signal für Leu und ein mittleres für Met. An den Positionen 3 bis 5 wurden jeweils 6 bis 8 Reste gefunden. Position 6 enthielt Val, Leu, Ile und Thr. Die folgenden zwei Positionen zeigten jeweils 3 Signale. Position 9 zeigte ein starkes Val- und ein schwaches Leu-Signal. Position 10 zeigte keinen Anstieg für einen Rest, was darauf hinweist, daß A2-restringierte Epitope Nonapeptide sind. Leu oder Met an Position 2 und Val oder Leu an Position 9 scheinen die Ankerreste zu sein. Einige von bekannten Peptiden mit A2-restringierten Epitopen können mit dem Motiv in Übereinstimmung gebracht werden, während dies bei anderen nur teilweise möglich ist (Tabelle 4c). Die Existenz von mehreren Varianten von A2-Molekülen kann diese schlechte Übereinstimmung einiger Peptide mit dem Motiv verursachen.

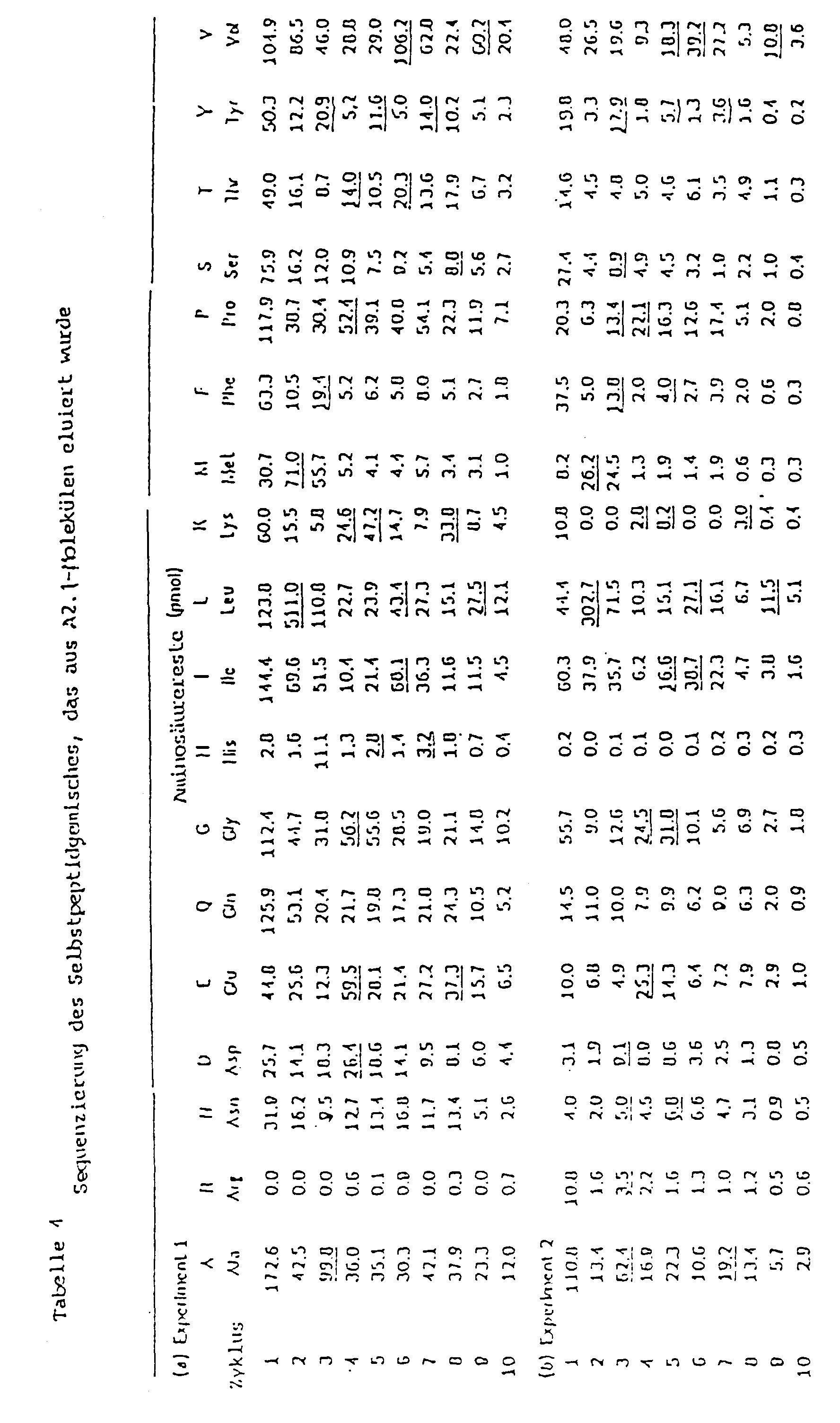

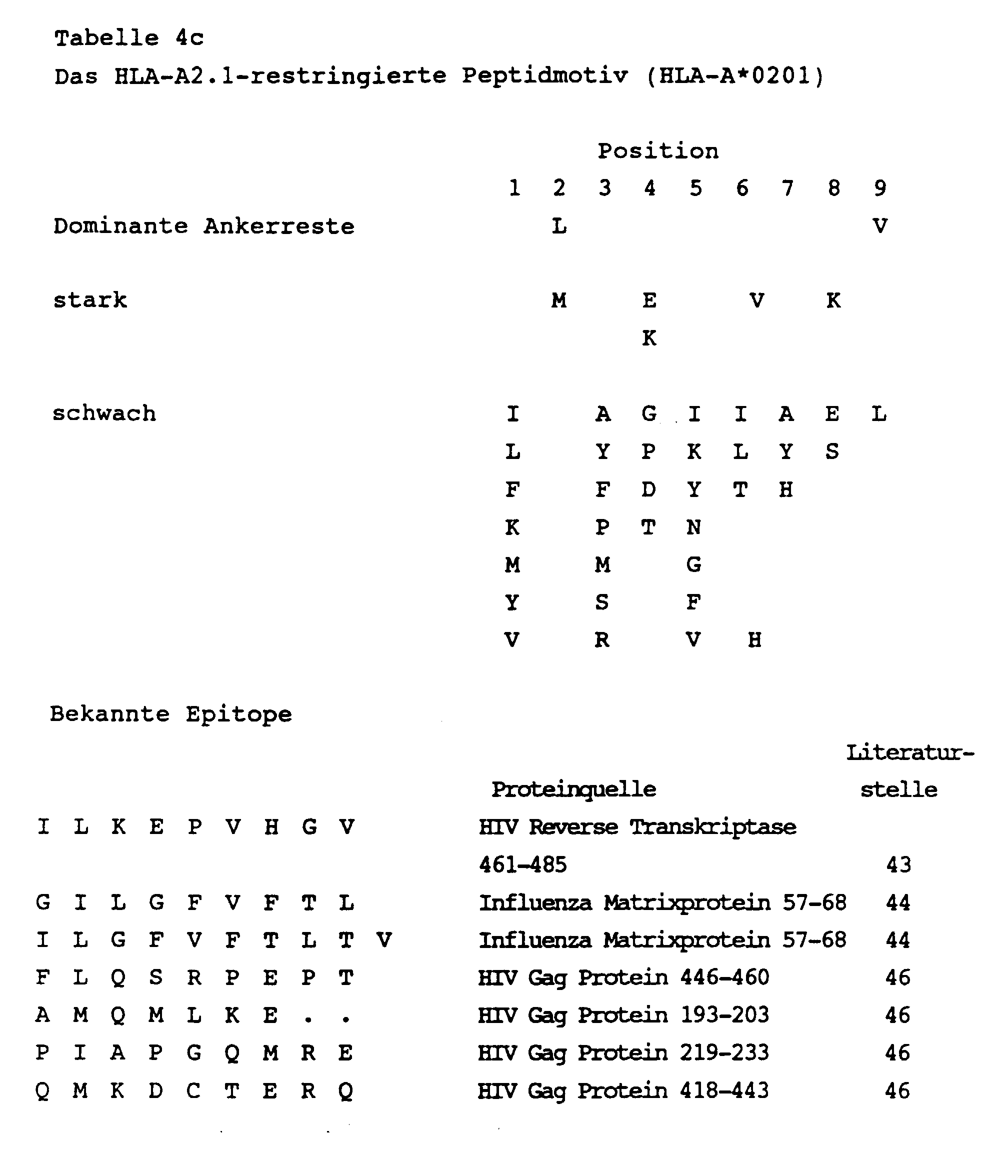

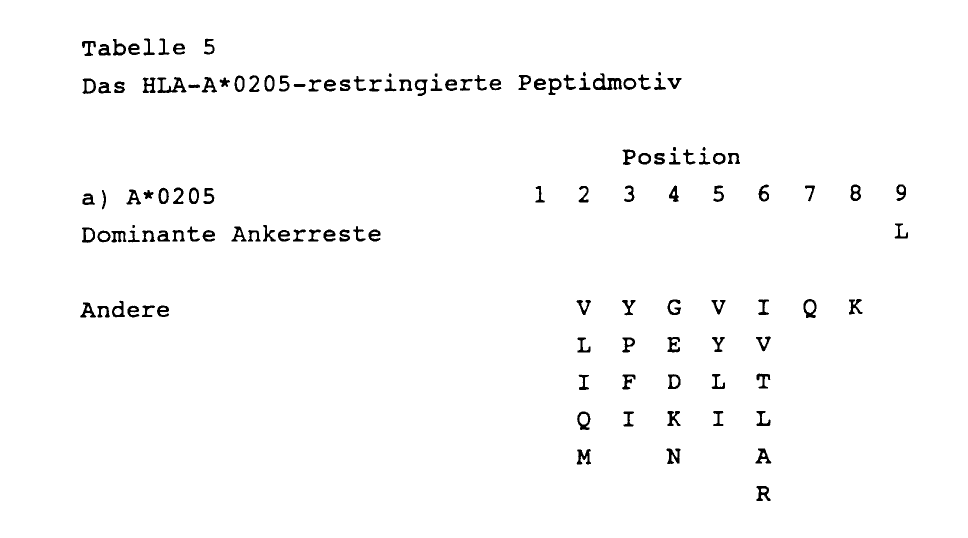

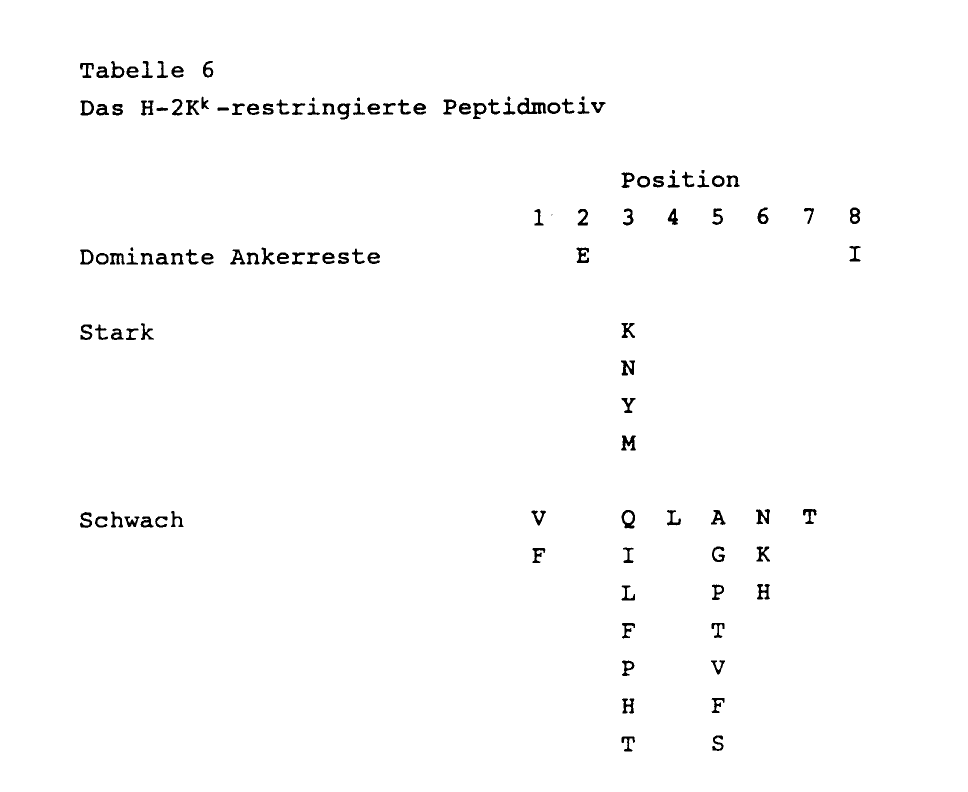

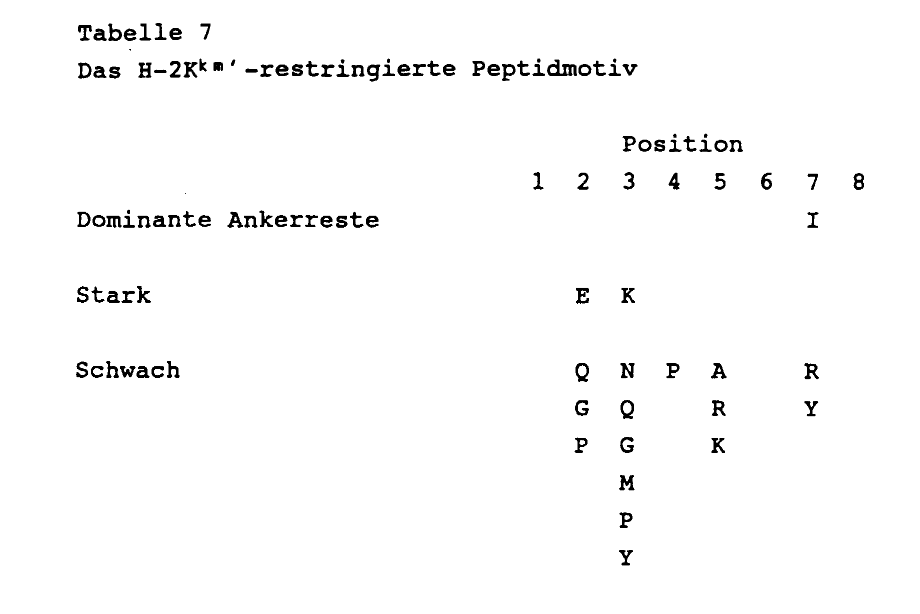

### Beispiel 4

Peptidmotive von HLA-A1, HLA-A3, HLA-A11, HLA-A24, HLA-A31, HLA-A33, HLA-B7, HLA-B8, HLA-B*2702, HLA-B*3501, HLA-B*3503, HLA-B37, HLA-B38, HLA-B*3901, HLA-B*3902, HLA-B*5101, HLA-B*5102, HLA-B*5103, HLA-B*5201, HLA-B58, HLA-B60, HLA-B61, HLA-B62, HLA-B78, HLA-Cw*0301, HLA-Cw*0401, HLA-Cw*0602, HLA-Cw*0702, HLA-Cw4, HLA-Cw6, HLA-Cw7, HLA-DRB1*0101, DRB1*1201, HLA-DR4w14, HLA-DR17, HLA-DRw52, HLA-DPw2, HLA-DPB1*0401, HLA-DQB1*0301, HLA-DQw1, HLA-DR1, HLA-DR3 und HLA-DR5

Die Bestimmung dieser Peptidmotive erfolgte entsprechend den Beispielen 1 bis 3. Die Ergebnisse sind in den Tabellen 8 bis 24 dargestellt.

Die Peptidmotive HLA-A, B und C sind MHC-Klasse I-Liganden. Die Peptidmotive HLA-DR, DQ und DP sind MHC-Klasse II-Liganden.

### Bemerkungen zur Epitopvorhersage für HLA-Klasse I-Liganden

Anker: Alle Anker werden in der Regel bei allen natürlichen MHC-Klasse I-Liganden benutzt. Jedoch kommt es auch vor, daß andere als die angegebenen Ankerreste, nämlich solche mit ähnlichen Eigenschaften, benutzt werden (z.B. kann eine hydrophobe Aminosäure durch eine andere hydrophobe ersetzt werden). Die Zahl der Aminosäuren zwischen den Ankern ist in der Regel konstant, jedoch kommt es auch vor, daß ein oder zwei weitere Aminosäuren eingeschoben sind.

Hilfsanker: Diese werden bevorzugt, jedoch nicht obligatorisch, benutzt; sind mehrere Hilfsanker angegeben, wird in der Regel zumindest ein Teil davon benutzt.

Bei der Epitopvorhersage bezüglich einer Proteinsequenz wird man zweckmäßigerweise folgendermaßen vorgehen:

Die Proteinsequenz wird nach Ankerresten im richtigen Abstand abgesucht. Die so gefundenen Teilsequenzen (Liganden-Kandidaten) werden auf das Vorhandensein von Hilfsankerresten an der richtigen Position geprüft, was die Zahl der Ligandenkandidaten einengt. Aus den verbliebenen Kandidaten werden die ausgewählt, die noch weitere der bevorzugten Aminosäurereste enthalten.

### Bemerkungen zur Epitopvorhersage für HLA-Klasse II-Liganden

Anker: Die HLA-Klasse II-Motive weisen 3 oder 4 Anker auf. Die einzelnen Liganden benutzen jedoch oft nur 2 dieser Anker. Vermutlich benutzen die besonders stark bindenden Liganden alle Anker, und vermutlich können fehlende Ankerreste durch Übereinstimmung in anderen bevorzugten Resten kompensiert werden.

Um dies zu verdeutlichen, sind bei den Ligandenbeispielen die passenden Ankerreste doppelt, die anderen bevorzugten Reste einfach unterstrichen.

Die allel-spezifischen Motive in den Tabellen 39 - 47 sind in relativen Positionen (Erster Anker = Relative Position 1) angegeben, da die Zahl der Aminosäurereste zwischen N-Terminus und erstem Anker bei Klasse II-Liganden variabel ist (im Gegensatz zu Klasse I-Liganden). In den Tabellen 48 - 50 sind die Motive in den absoluten Positionen angegeben.

Die Vorgehensweise bei der Epitop-Vorhersage innerhalb einer Proteinsequenz wird ähnlich sein wie bei Klasse I, nur daß von vornherein die Sequenz nach Übereinstimmung mit mindestens 2 Ankerresten (davon einer Anker 1) abgesucht wird. Werden auf diese Weise mehrere Ligandenkandidaten erhalten, werden zur weiteren Einengung die anderen bevorzugten Reste verglichen und schließlich auf Übereinstimmung mit dem (nicht allel-spezifischen) Prozessierungsmotiv (Protein aus absoluter Position 2 bzw. 12 bis 16) geprüft.

In der absoluten Position 2 der untersuchten HLA-Klasse II-Liganden findet sich ein sehr starkes Pro-Signal. Weitere Pro-Signale finden sich im Bereich des C-Terminus. Diese Pro-Signale scheinen ein bevorzugtes Merkmal von natürlichen Klasse II-Liganden zu sein. Das Prozessierungsmotiv für HLA-Klasse II-Liganden ist daher wie folgt:

| absolute Postion | | | | | | | | | | | | | | | | |
|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 |
| | P | | | | | | | | | | p | p | p | p | p | |

In den Tabellen 39 - 47 liegt der erste Anker im allgemeinen im Bereich der absoluten Positionen 3 - 5 oder 4 - 6.

Der obere Teil von Figur 2 zeigt einen vereinfachten Vergleich von Klasse II- und Klasse I-Liganden. Klasse II-Liganden (links) sind in der MHC-Spalte durch allel-spezifische Taschen verankert. Beide Enden der Spalte sind offen, d.h. die Liganden (12 - 25 Reste, durchschnittlich 15 - 16 Reste) können heraushängen. Der zweite Rest nach dem N-Terminus ist häufig Pro, vermutlich als Ergebnis einer Aminopeptidaseaktivität. Wie aus Figur 2 hervorgeht, ist dieser Pro-Rest nicht an der Bindung mit der MHC-Spalte beteiligt. D.h., ein synthetisches, MHC II-bindendes Peptidmotiv muß den Pro-Rest nicht enthalten, sondern es beginnt vorzugsweise erst mit dem ersten Ankerrest. Der Abstand zwischen den N-Termini und dem ersten Anker ist 5 ± 1 Reste für die Mehrzahl der Liganden. Der Abstand zwischen dem letzten Anker und dem C-Terminus ist nicht konstant. Die Hauptunterschiede zu Liganden der Klasse I (rechts) sind die feste Bindung der Peptidtermini innerhalb der Spalte und die besser definierte Länge der Liganden.

Der untere Teil von Figur 2 zeigt die hypothetische Bindung von Klasse II-Liganden an ihren Rezeptor. Der Ligand ist als Peptidrückgrat in einer langgestreckten Orientierung dargestellt. Der erste hydrophobe Anker ist am α-Ende der Spalte und der letzte am entgegengesetzten Ende. Der zweite Anker ist etwa in der Mitte, wo sich α- und β-Domänen treffen. Somit stimmt der Abstand zwischen dem ersten und dem letzten Anker mit der Länge der Spalte überein. Die relativ konservierten Charakteristiken des ersten Ankers (hydrophob/aromatisch) den unterschiedlichen Allelen können das Fehlen eines verstärkten Polymorphismus in den DNA-Genen wiederspiegeln, während der zweite und der letzte Anker dem Einfluß der polymorphen β-Ketten ausgesetzt sind.

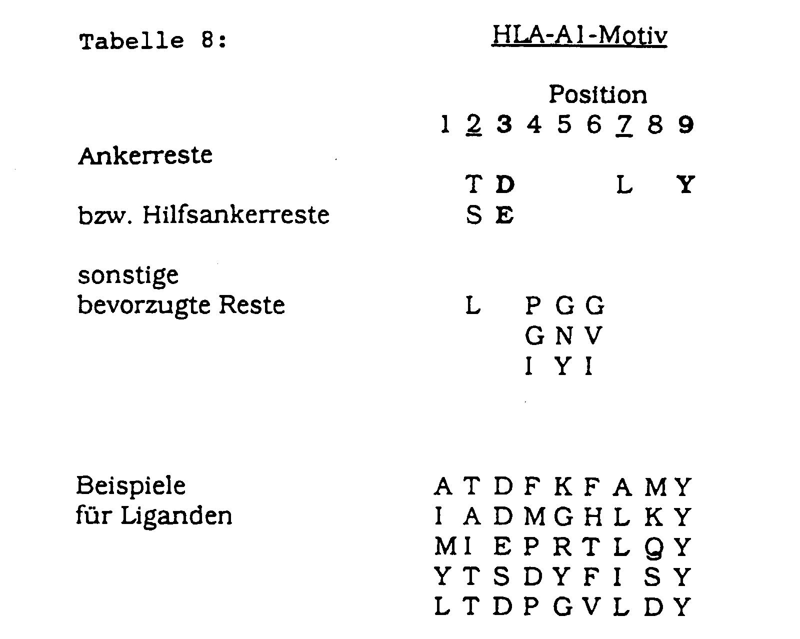

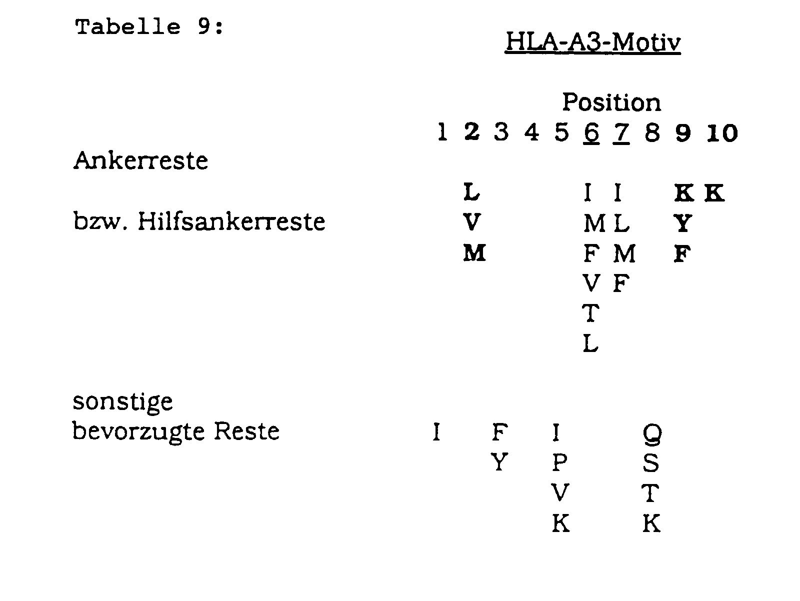

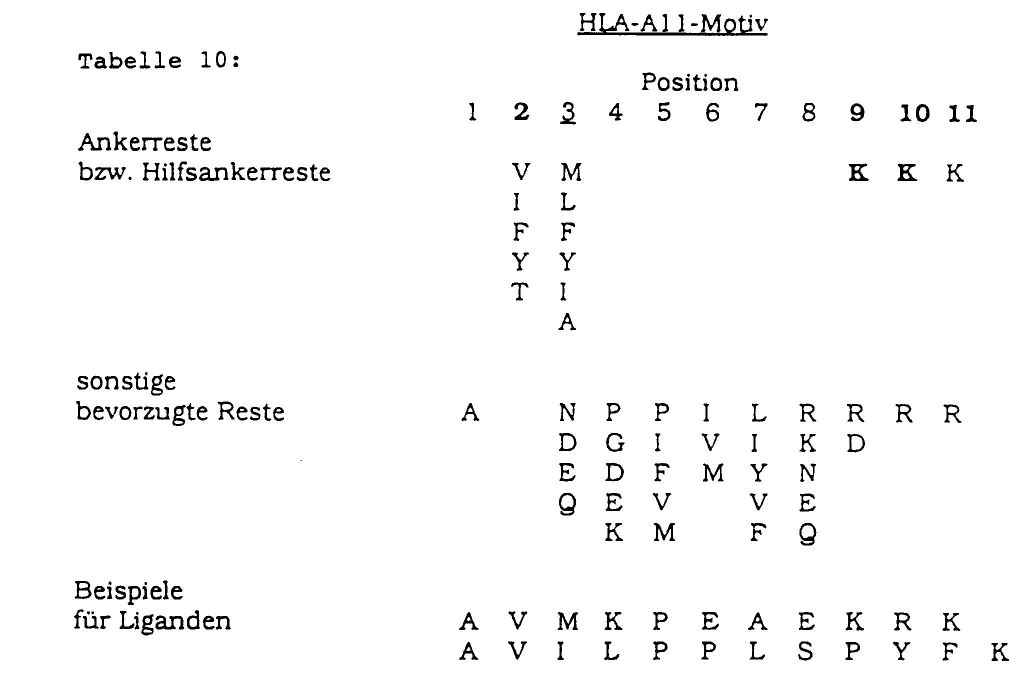

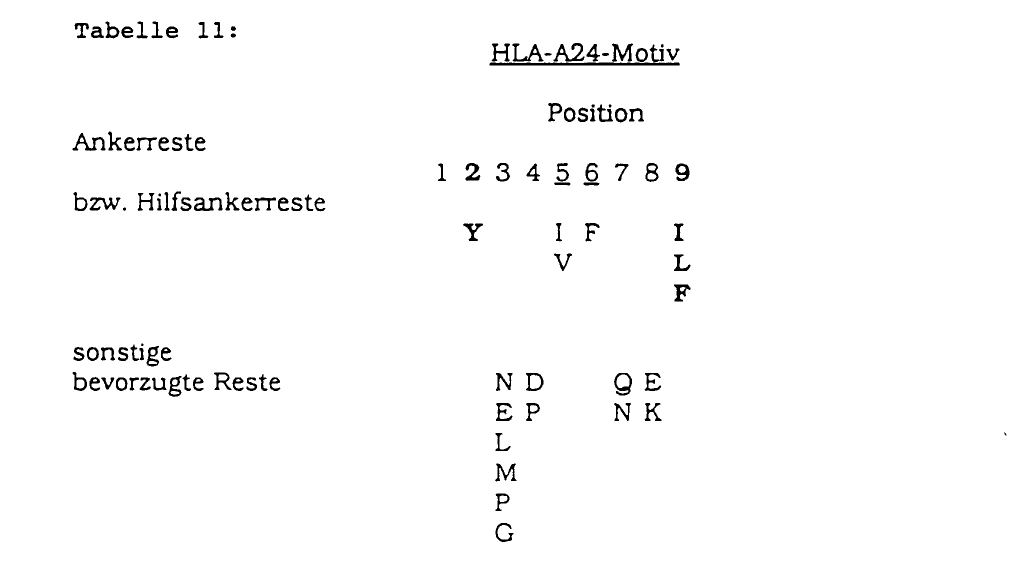

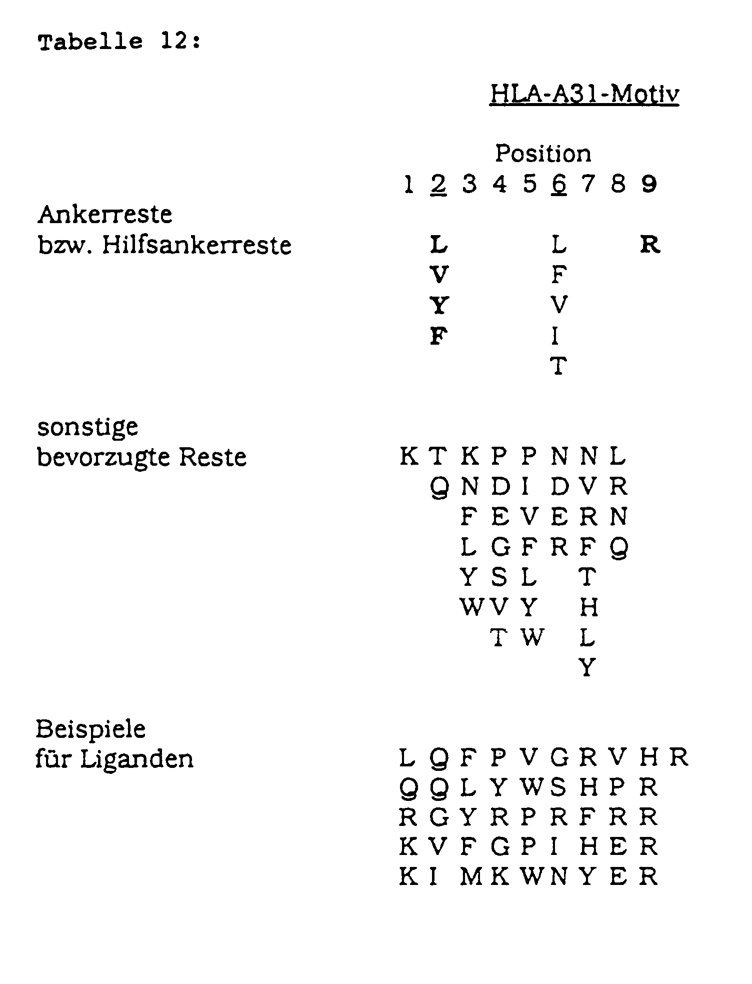

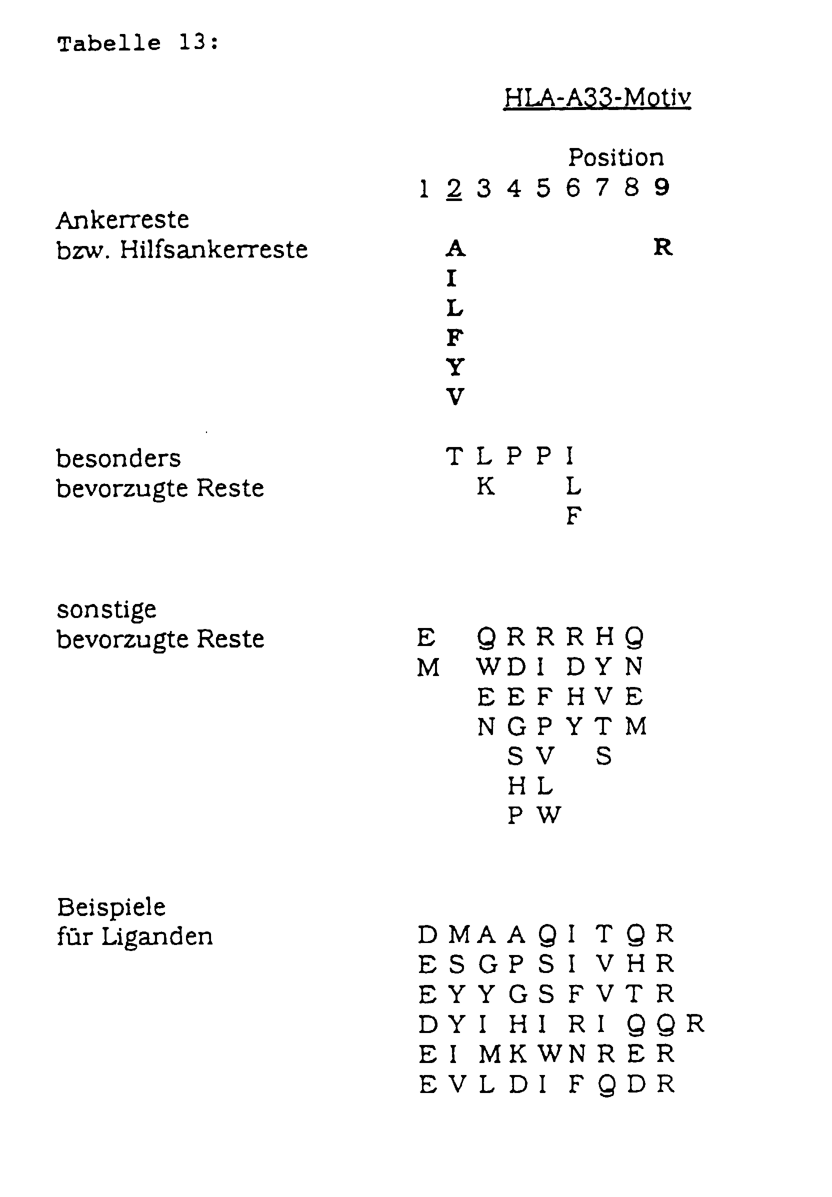

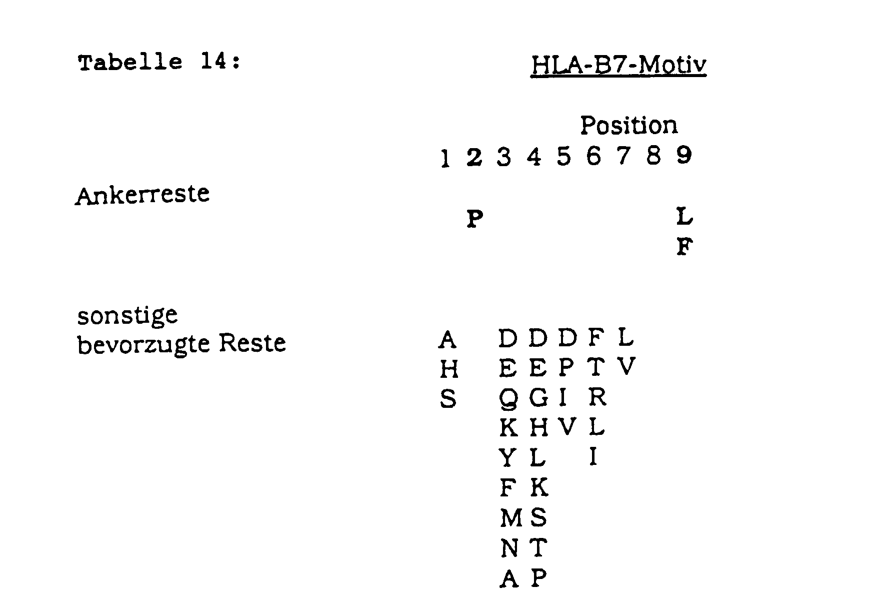

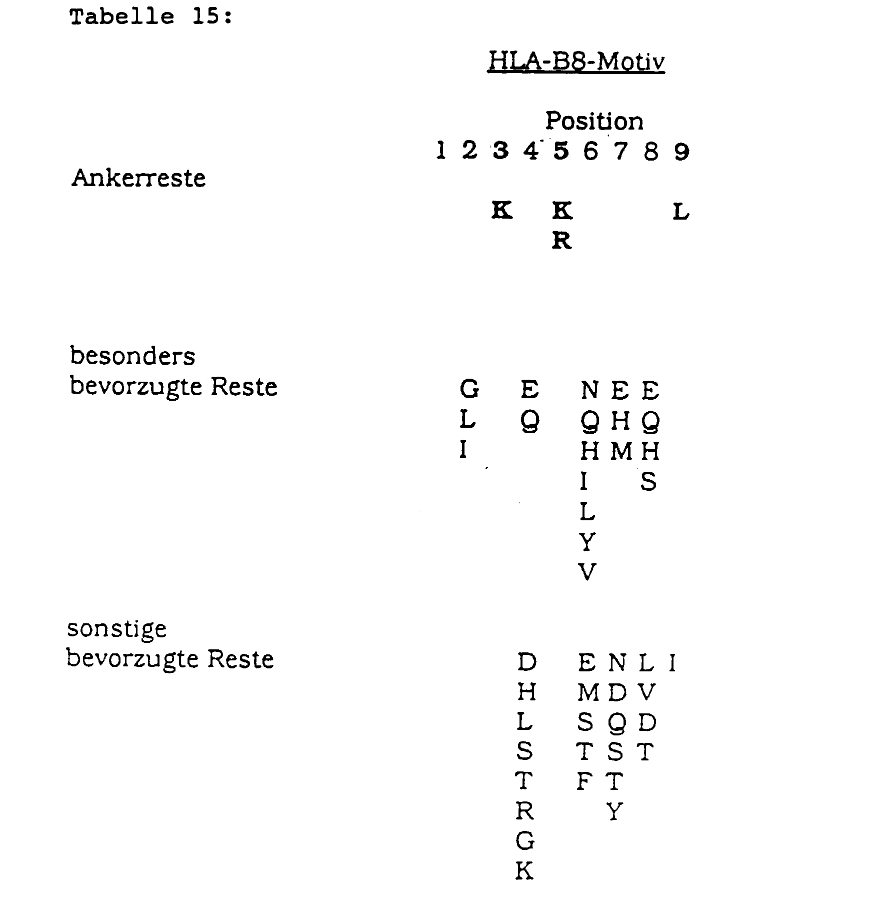

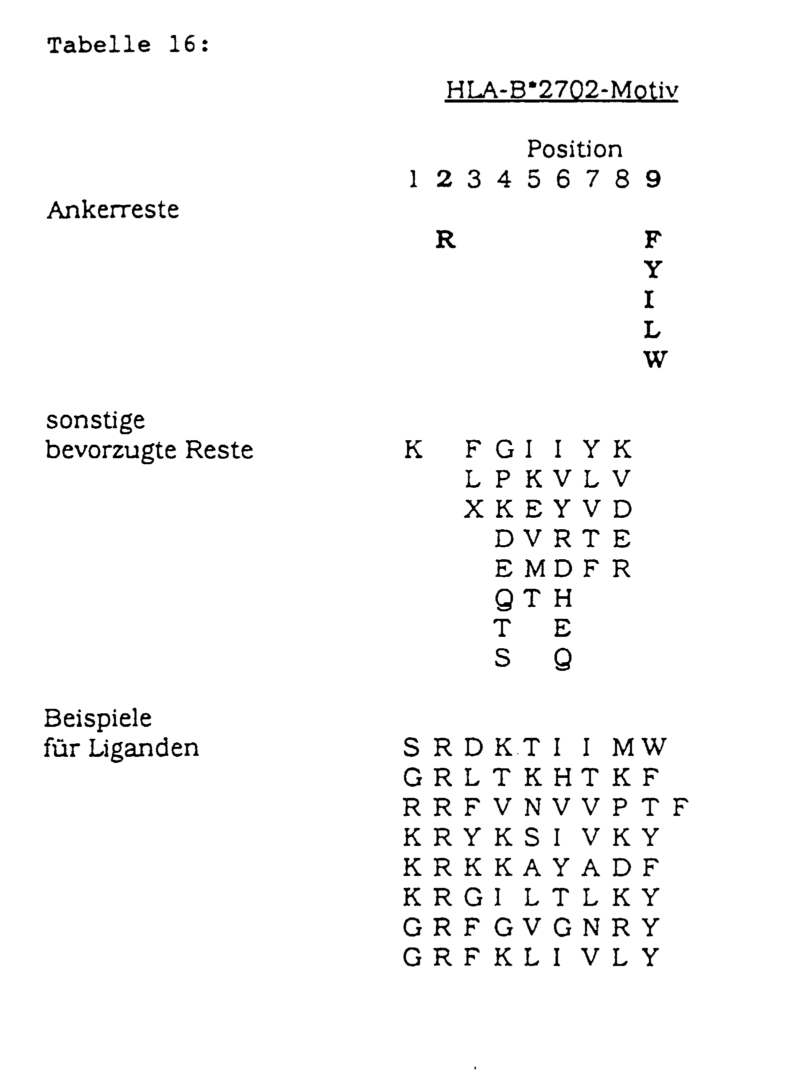

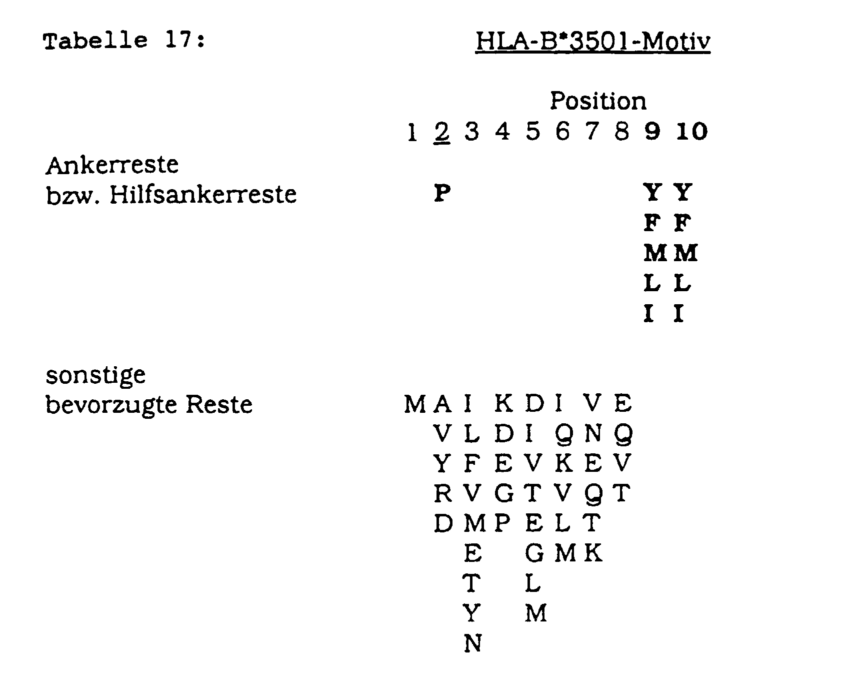

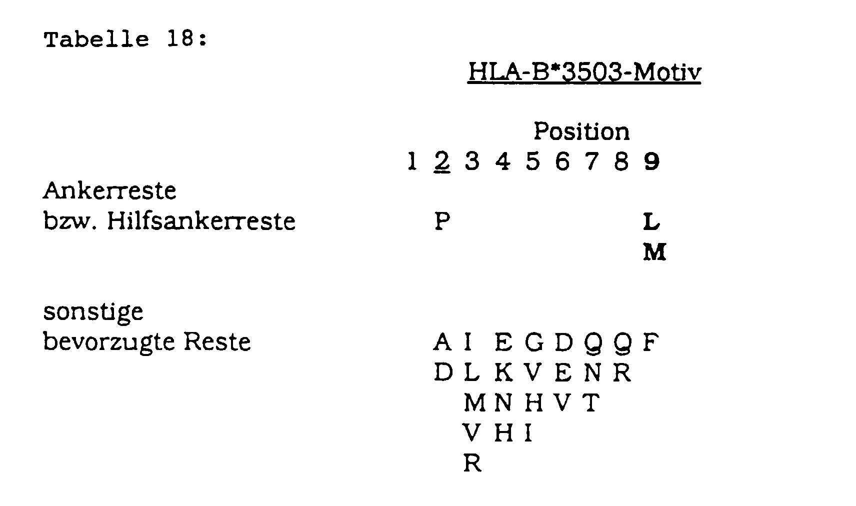

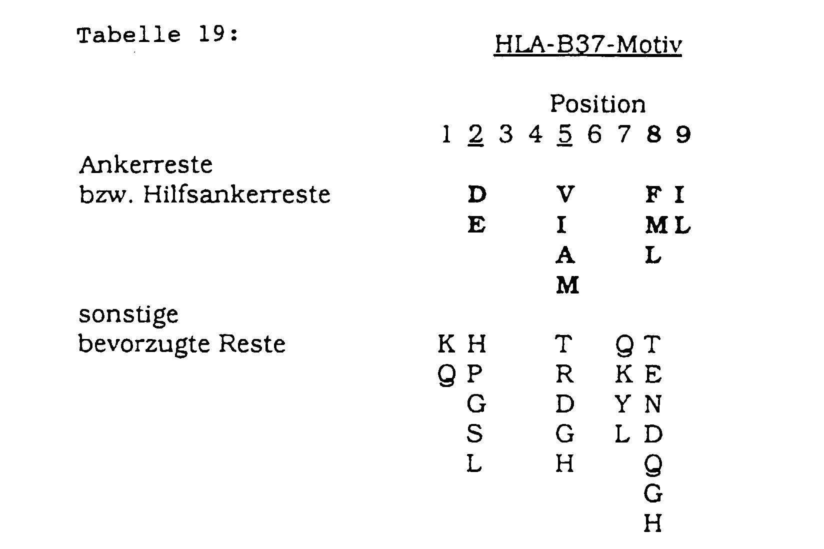

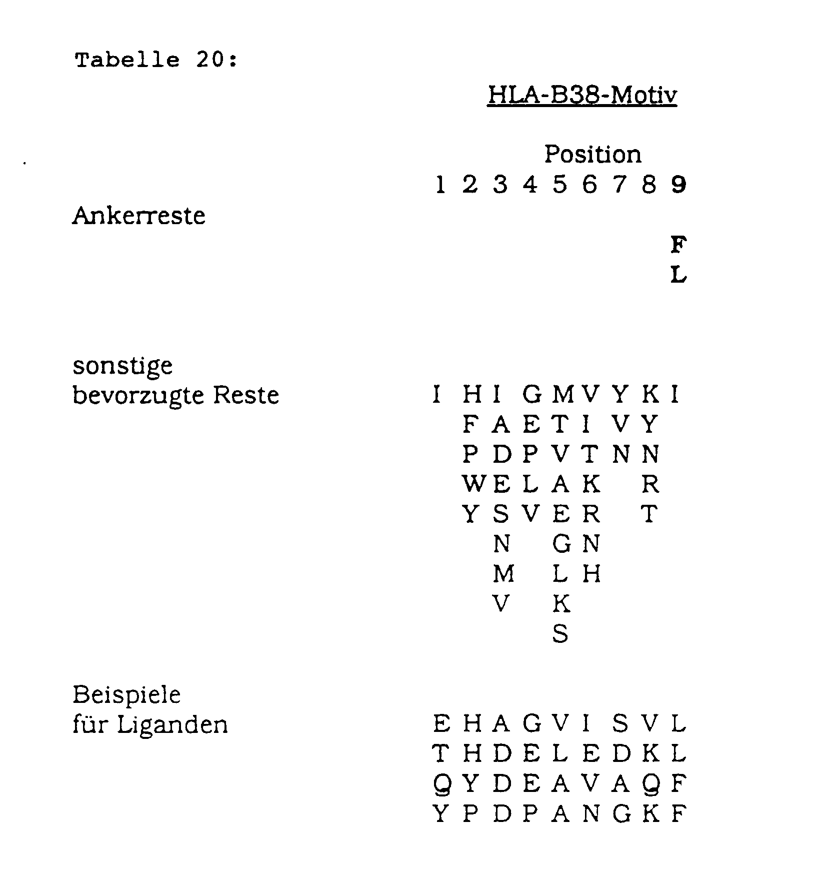

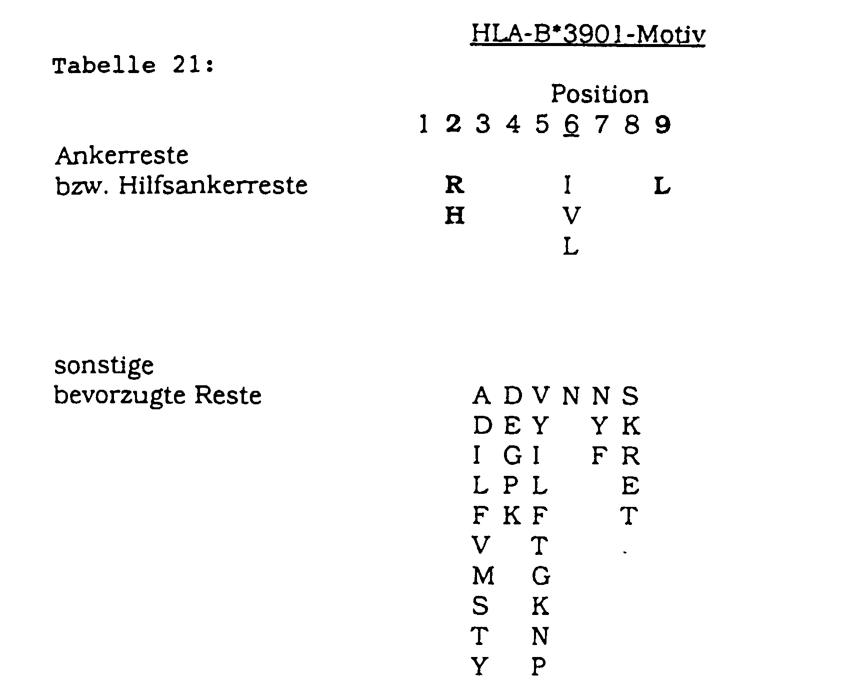

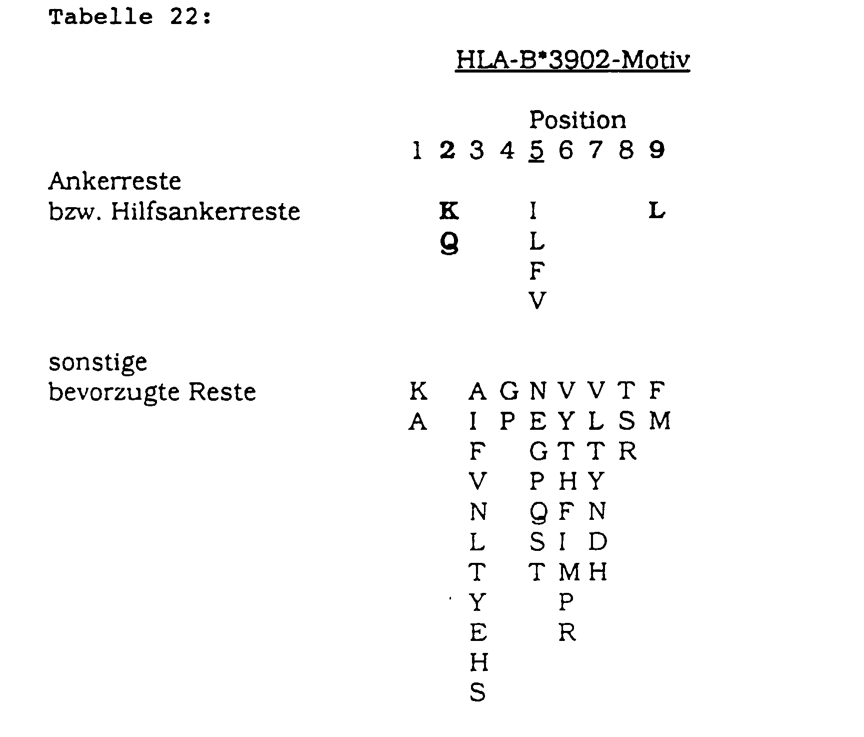

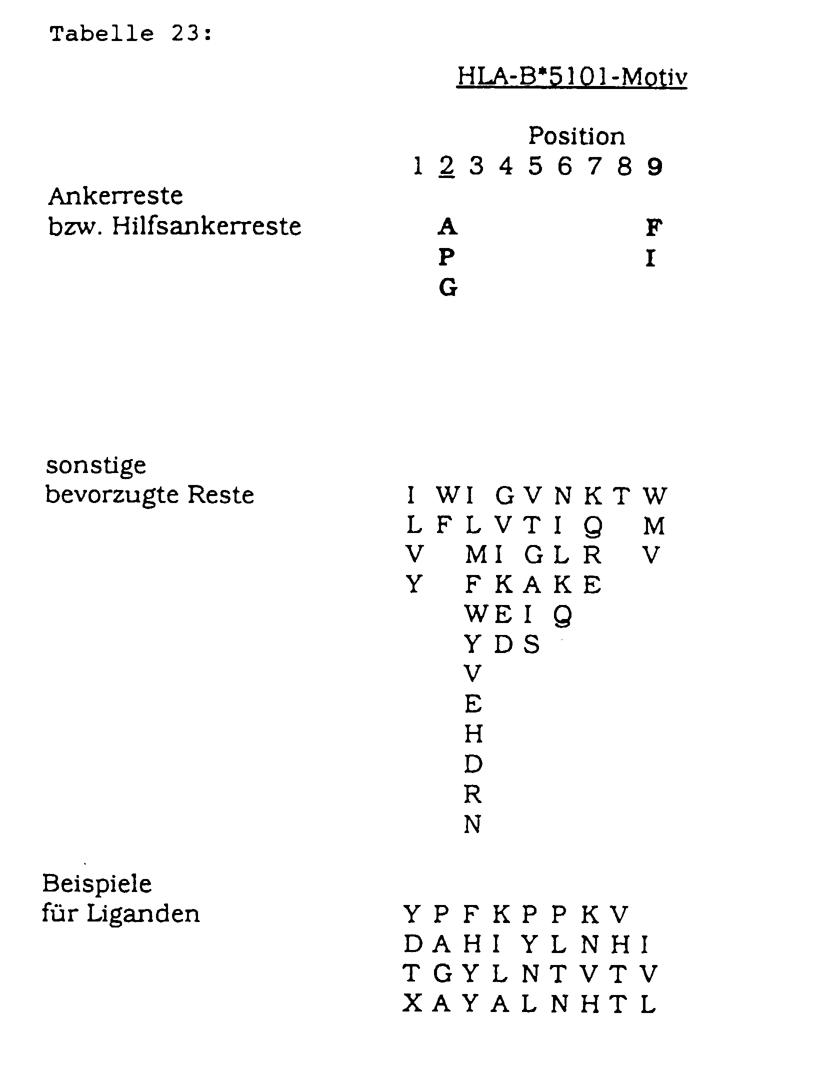

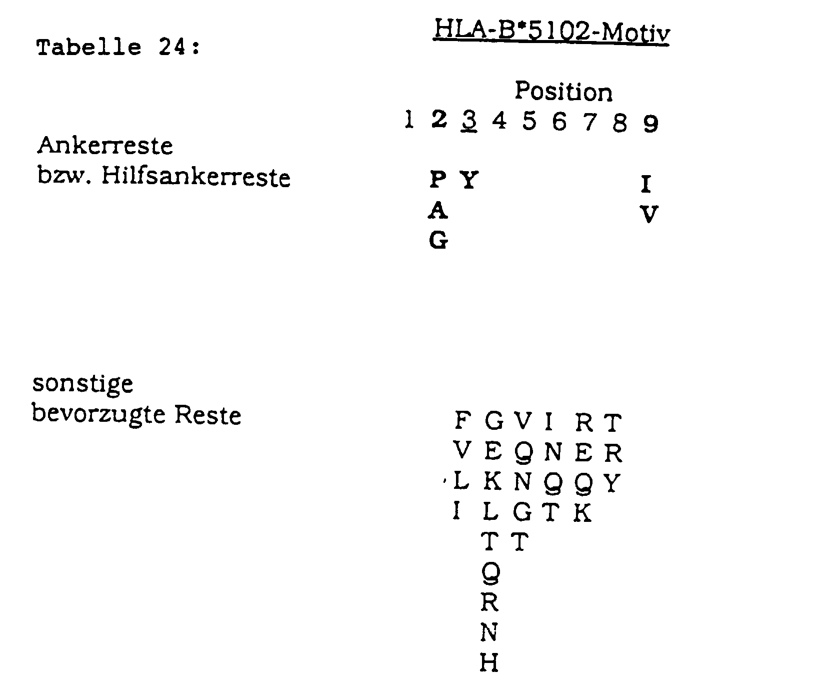

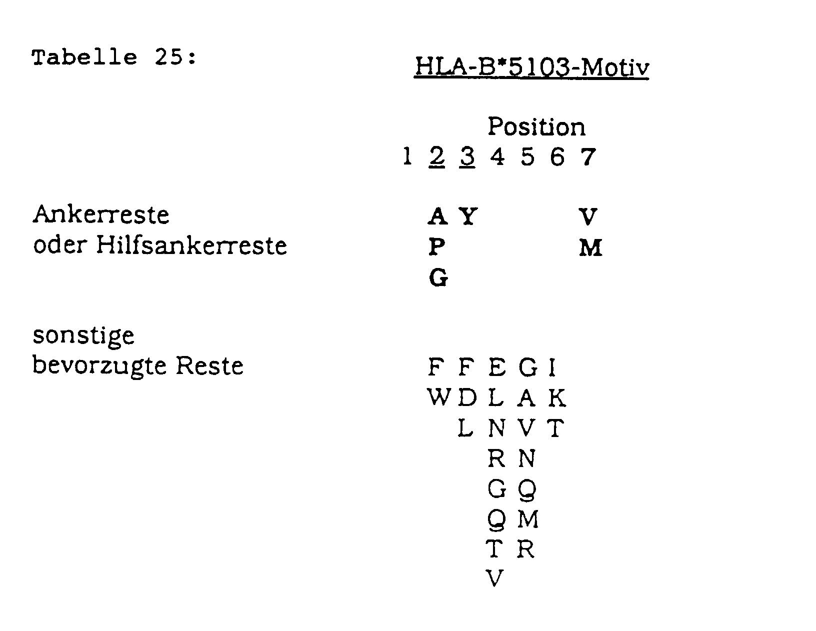

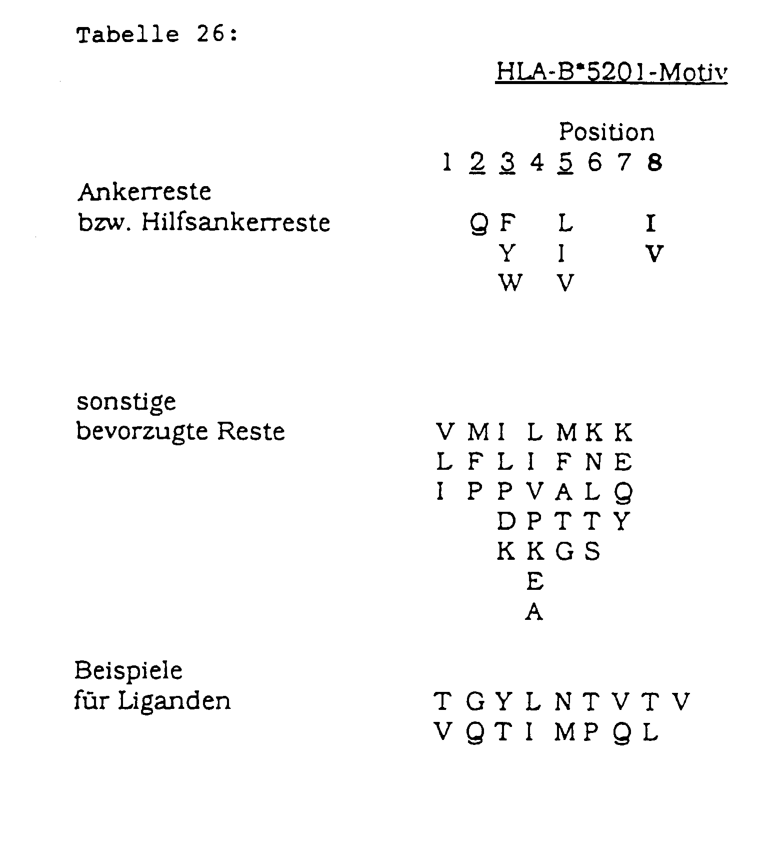

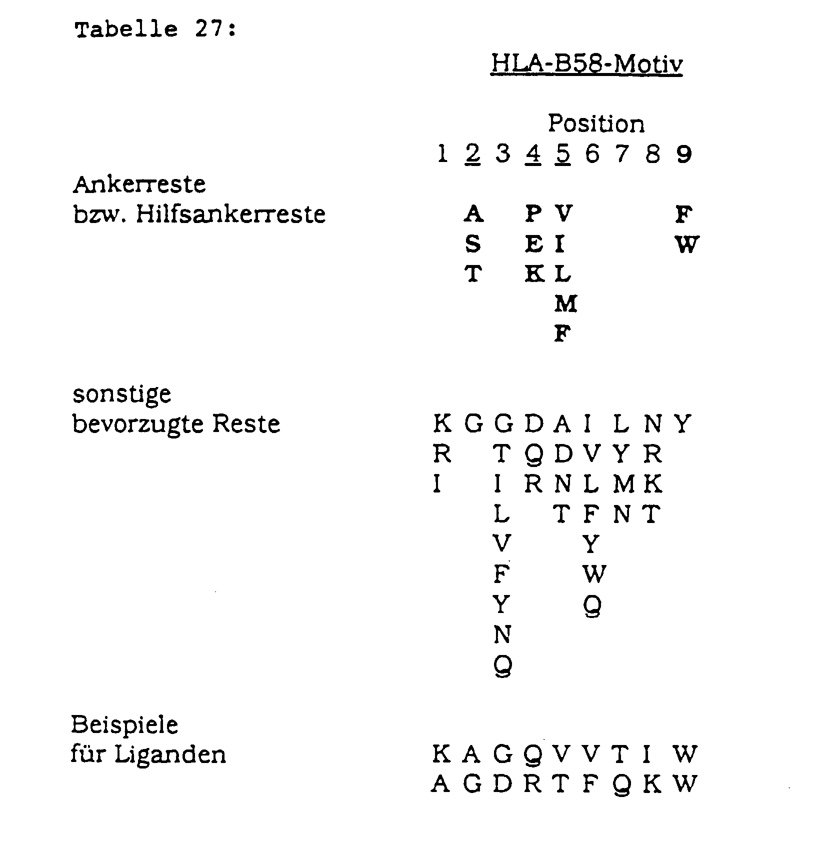

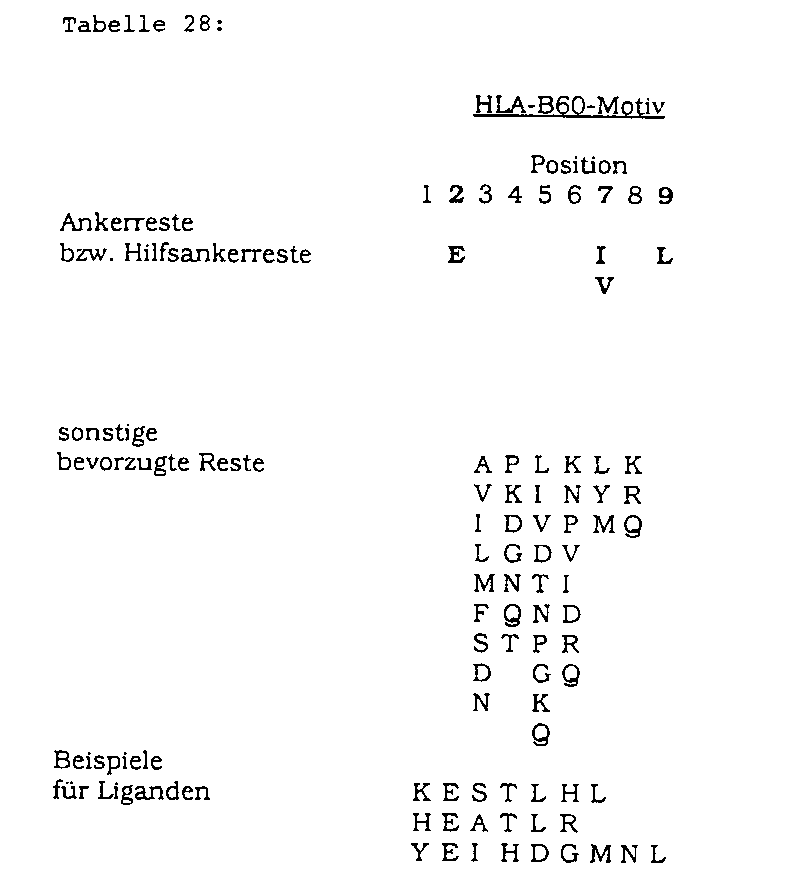

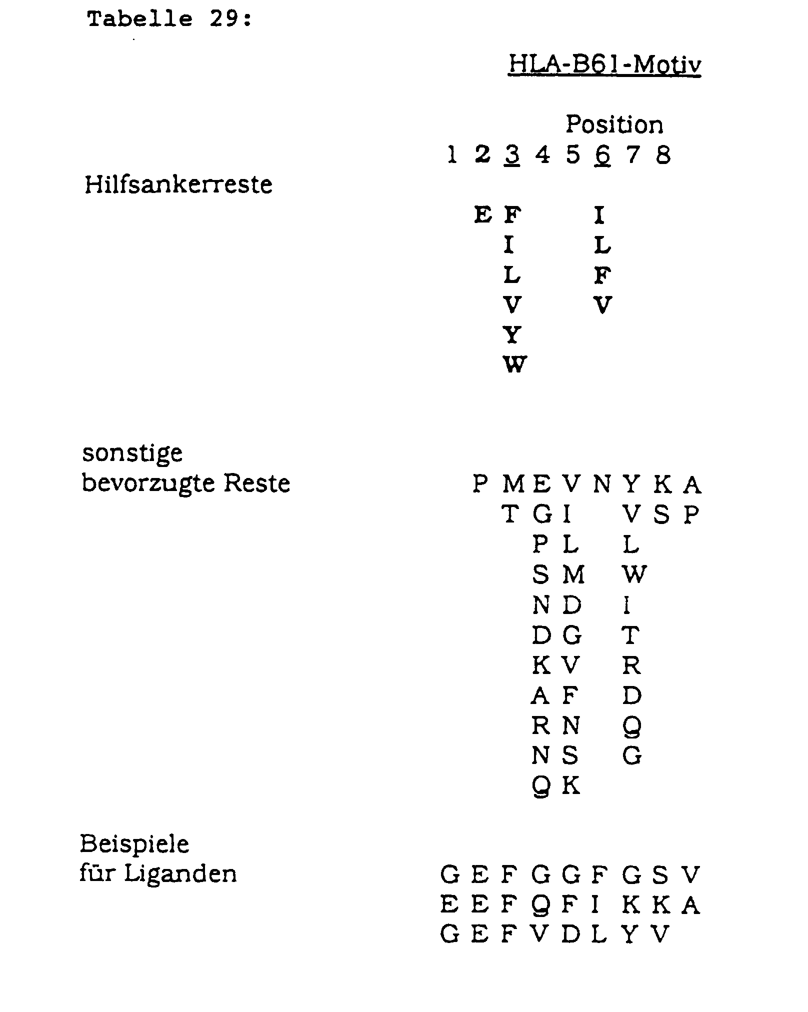

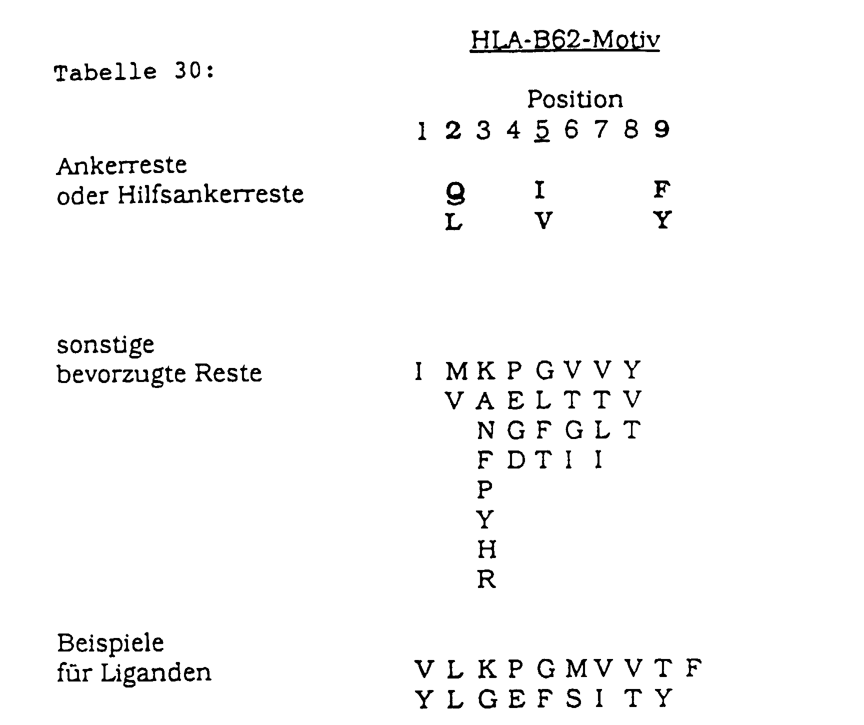

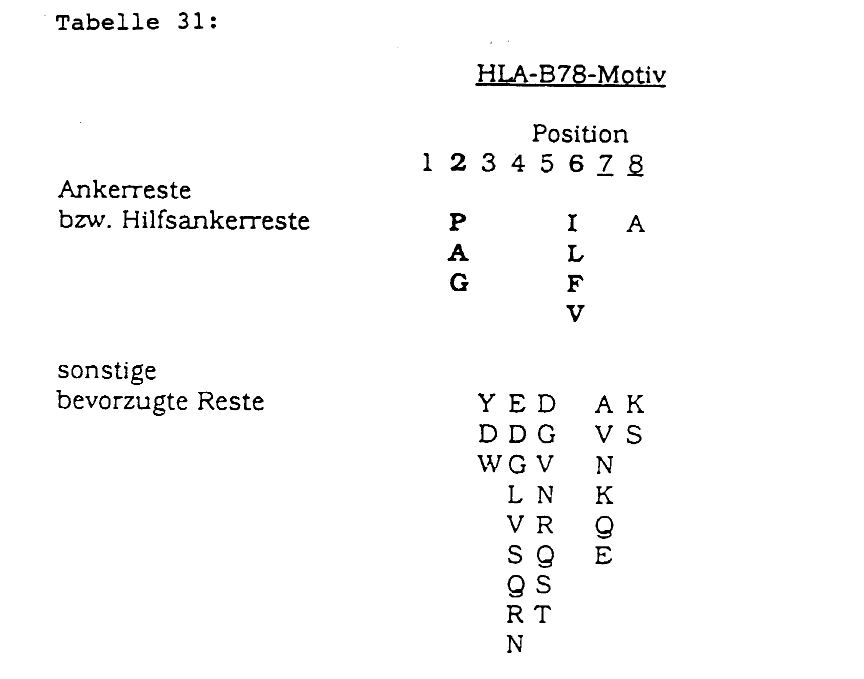

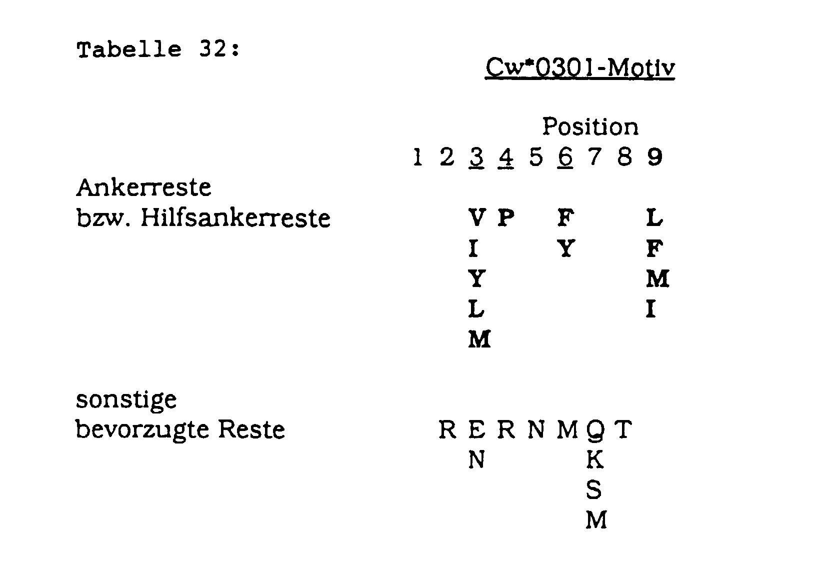

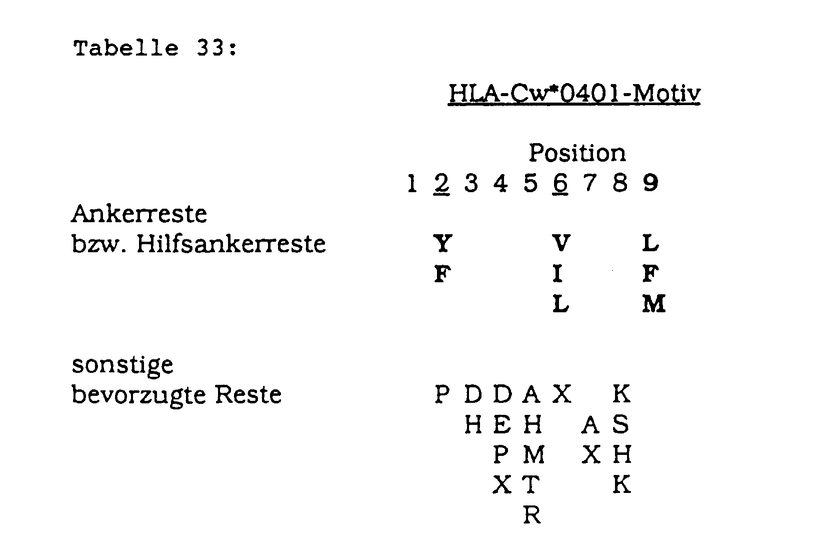

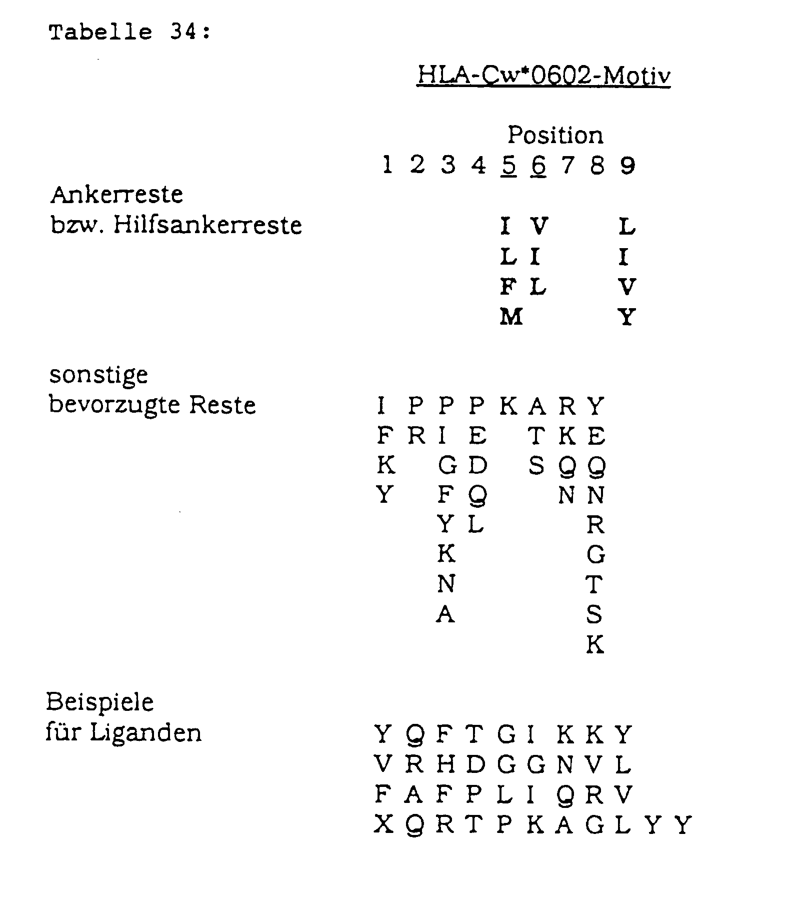

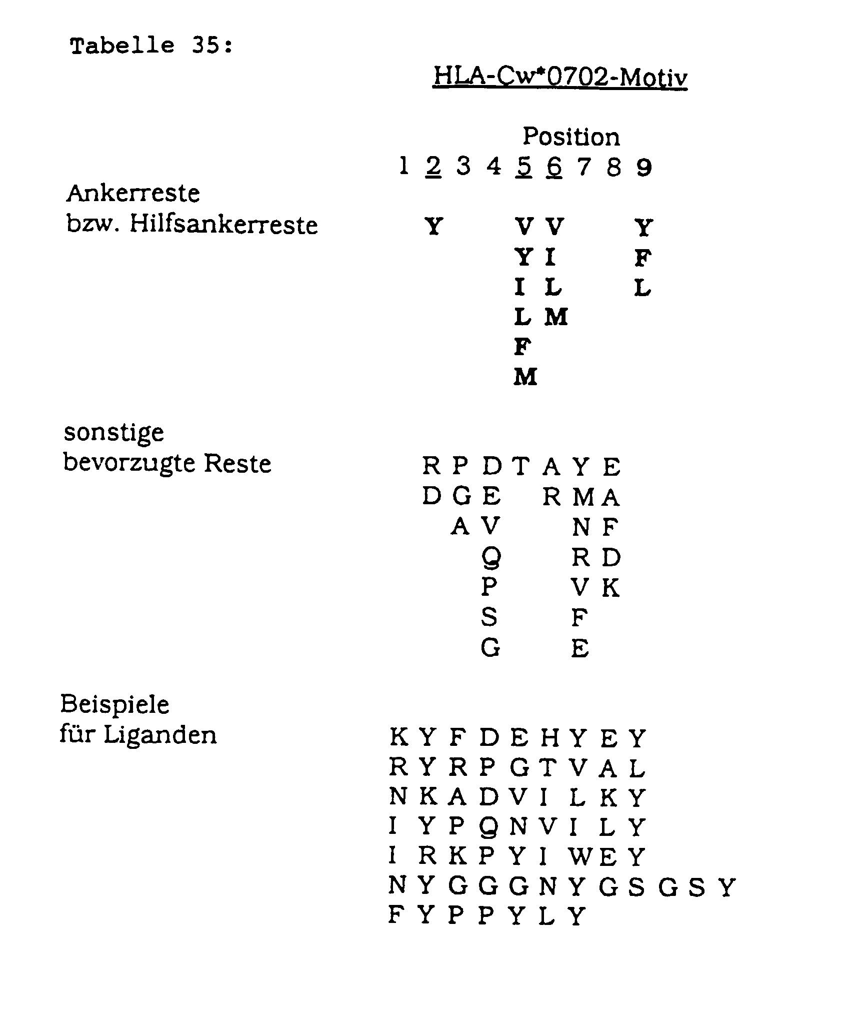

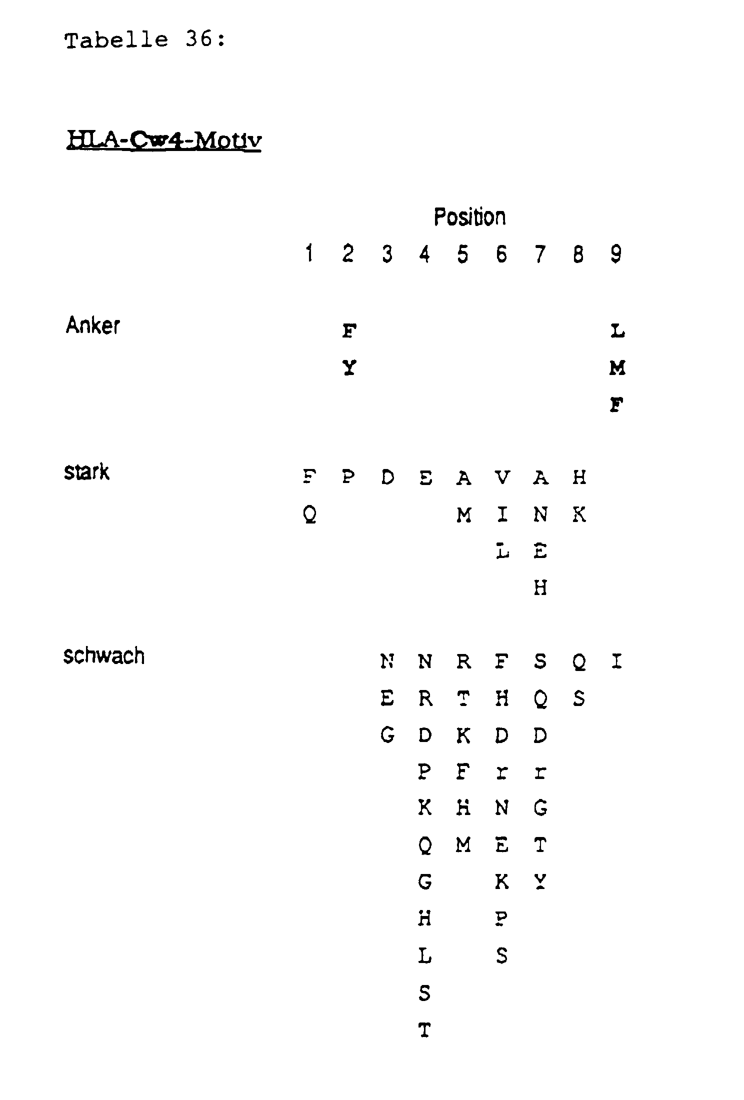

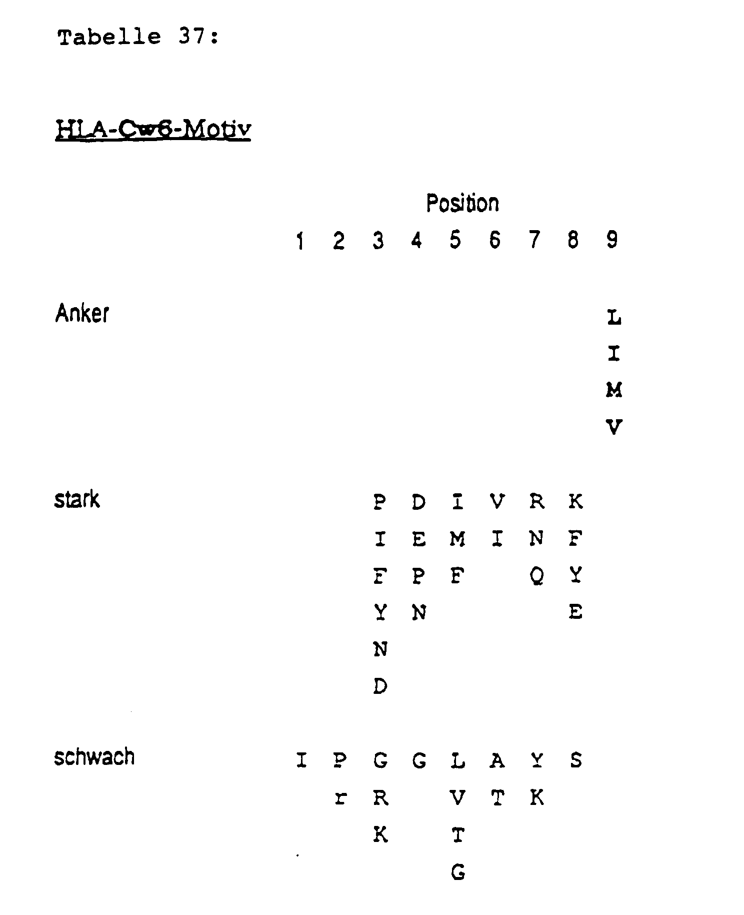

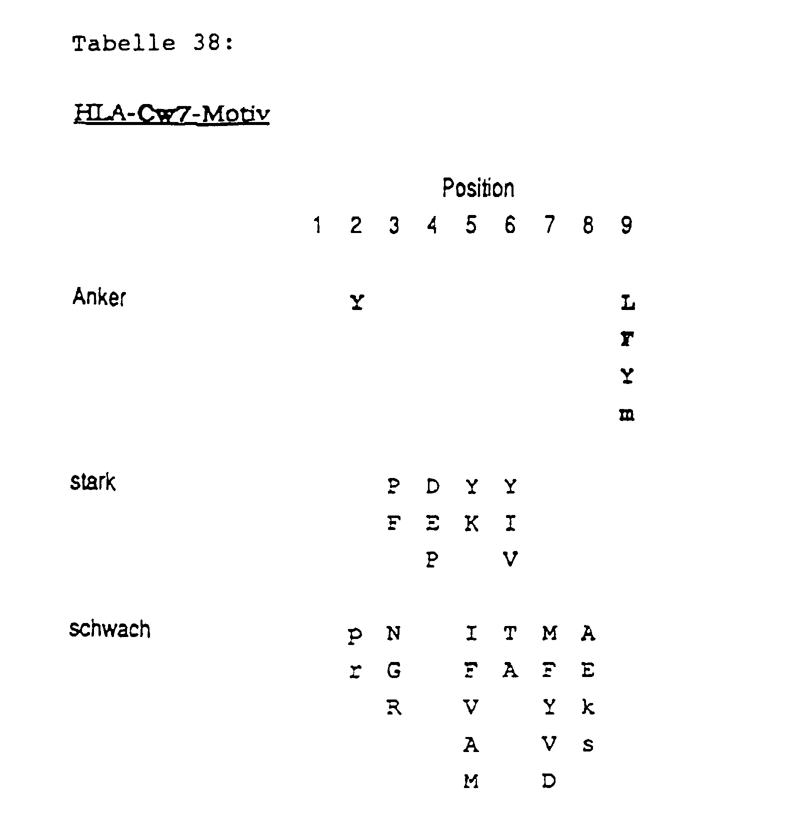

Demnach haben HLA- Cw4-. Cw6- und Cw7-Liganden folgende Eigenschaften:
1.) Überwiegende Länge von 9 Aminosäuren (längere und kürzere Peptide können vorkommen)
2.) Überwiegend aromatische Reste F oder Y an Position 2
3.) Überwiegend hydrophobe Reste V, I, L, F, A an Position 6 ("Hilfsanker")
4.) Hydrophobe Reste L, F, Y, M, V am C-Terminus
Individuelle Unterschiede der Liganden-Spezifität von Cw4, Cx6, und Cw7 gehen aus den Tabellcn hervor.

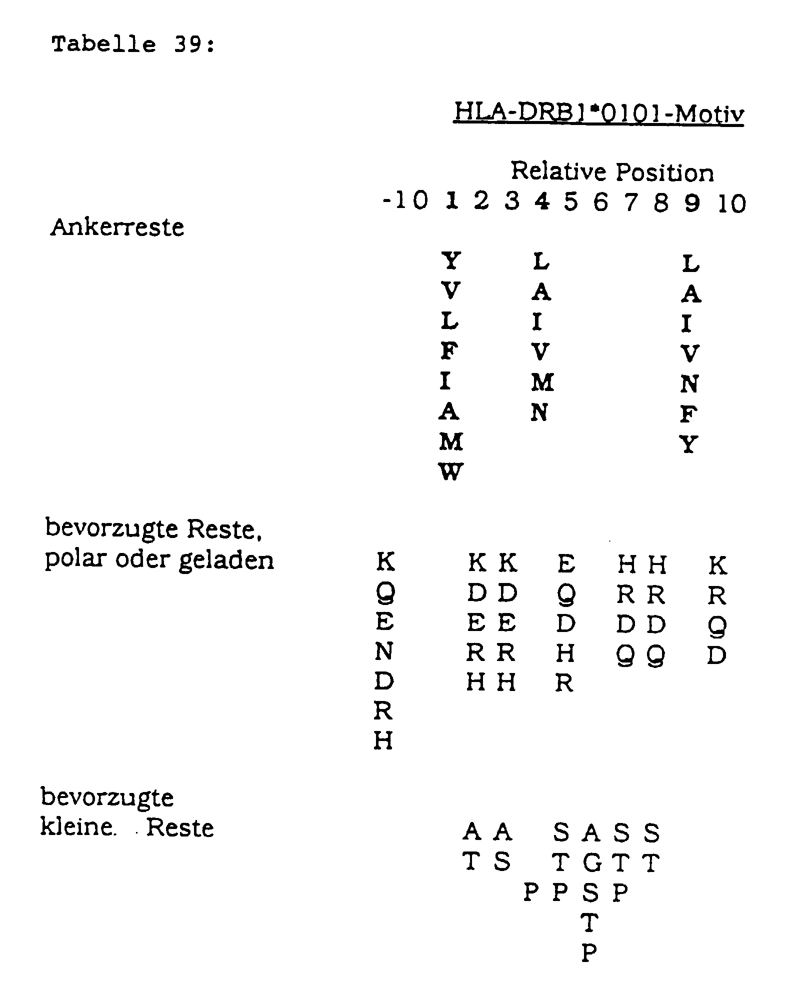

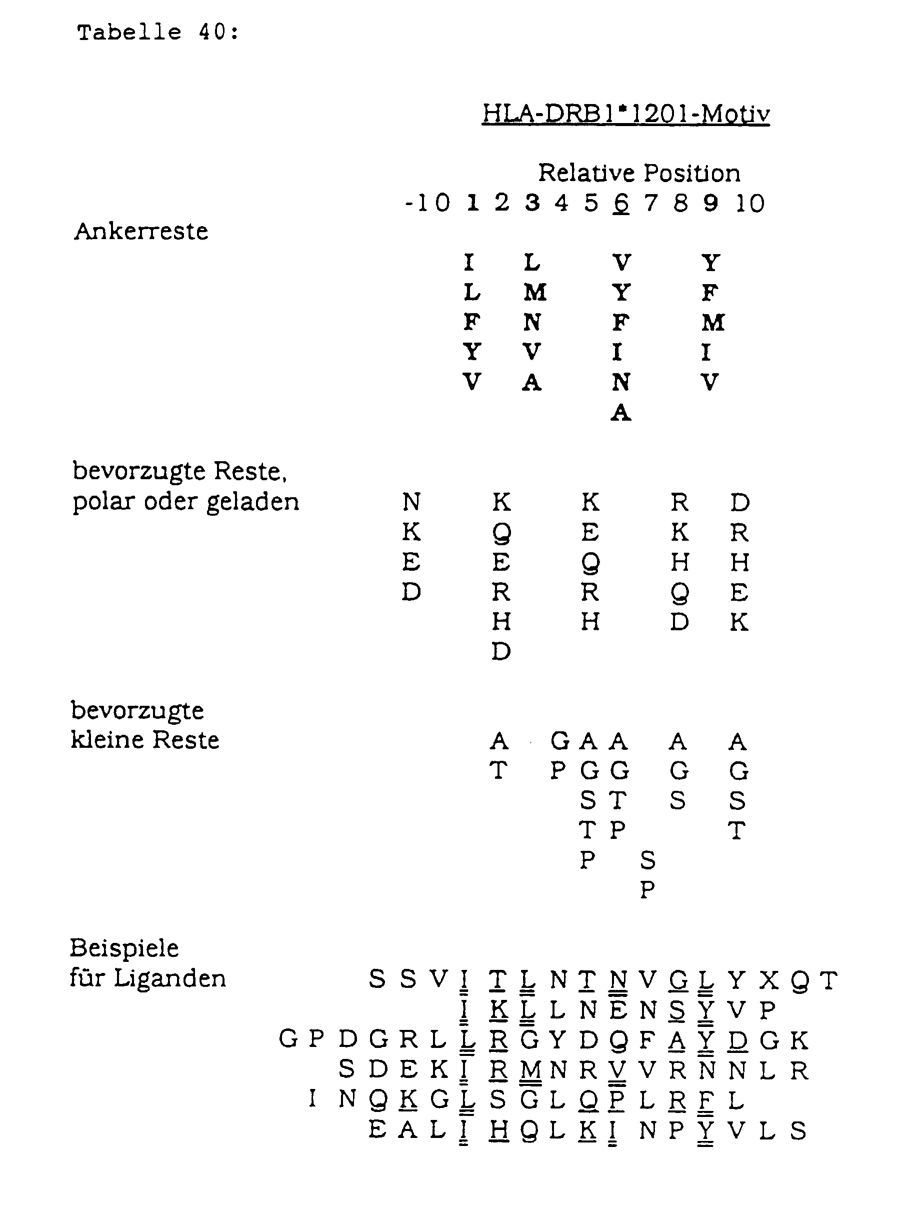

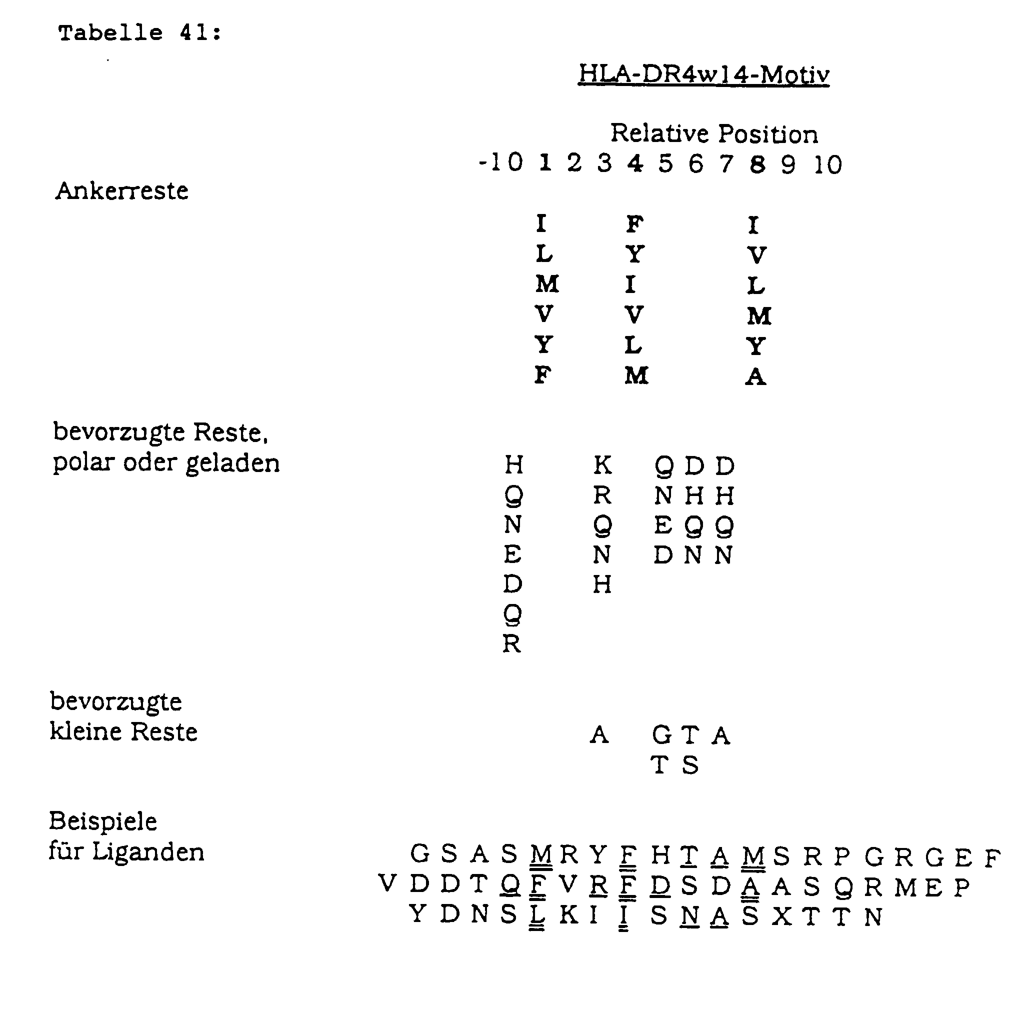

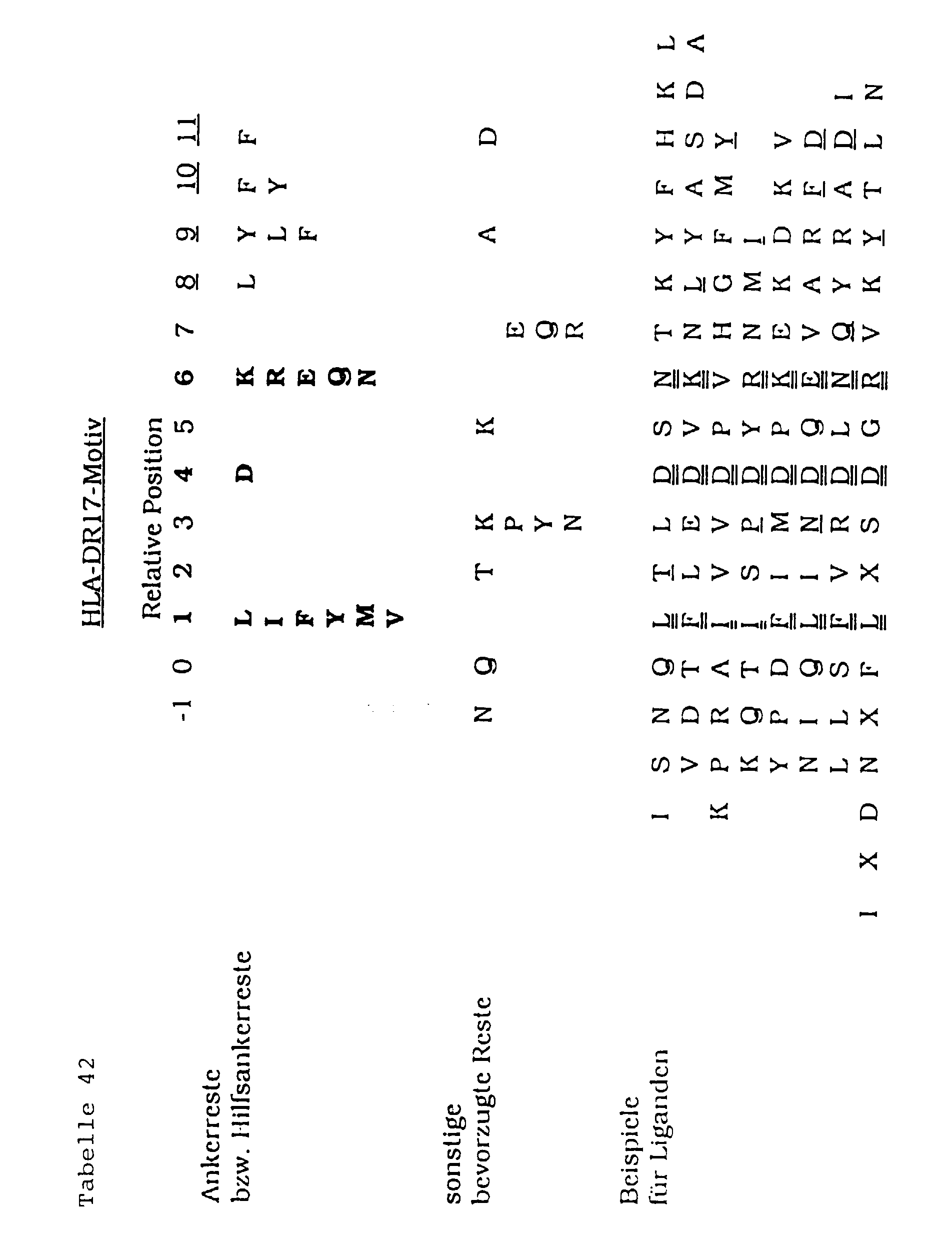

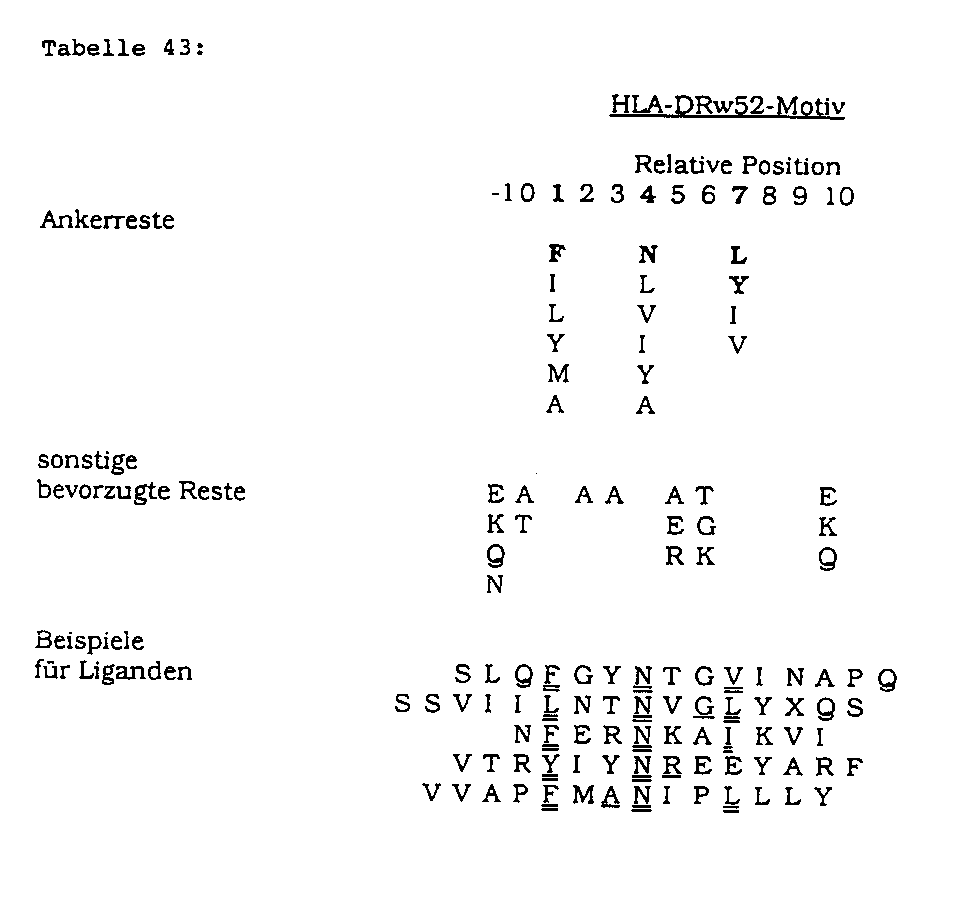

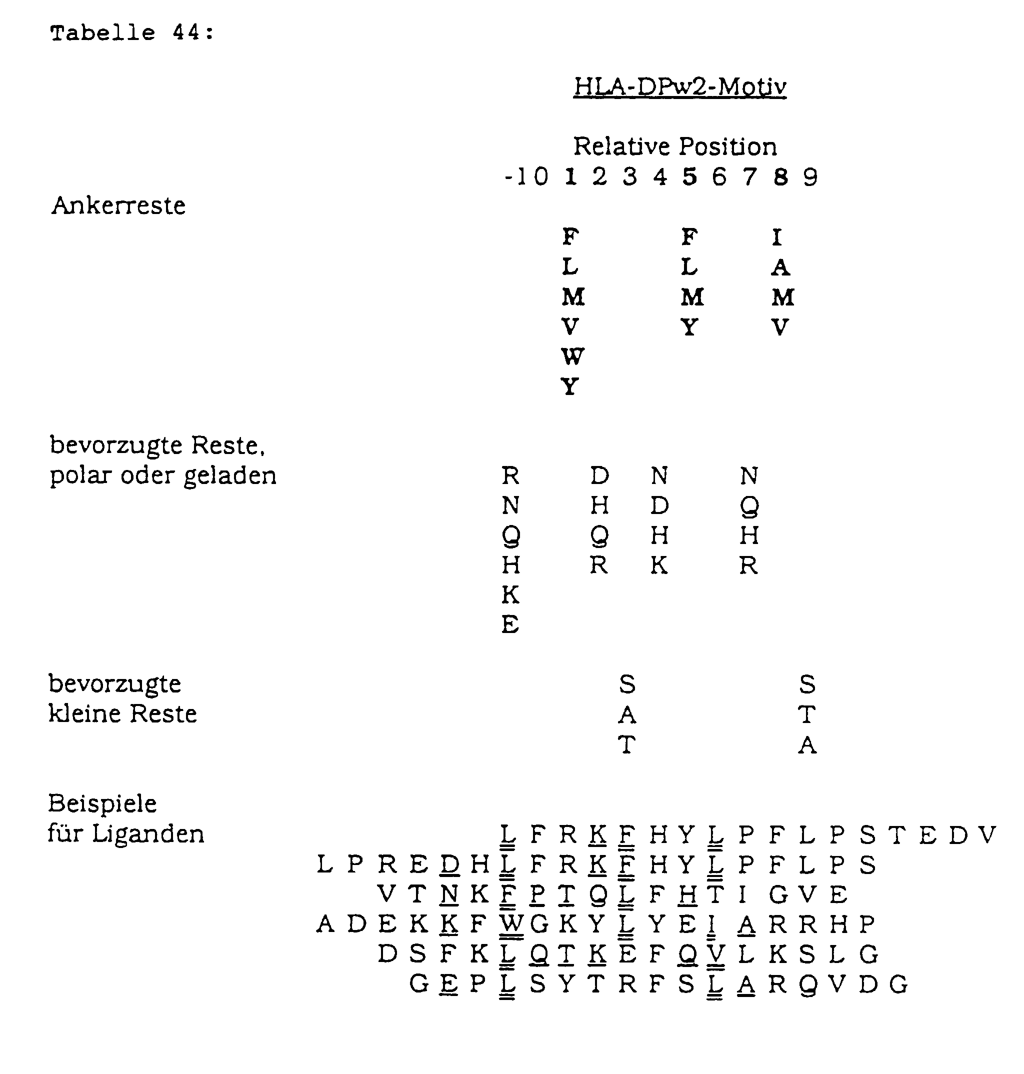

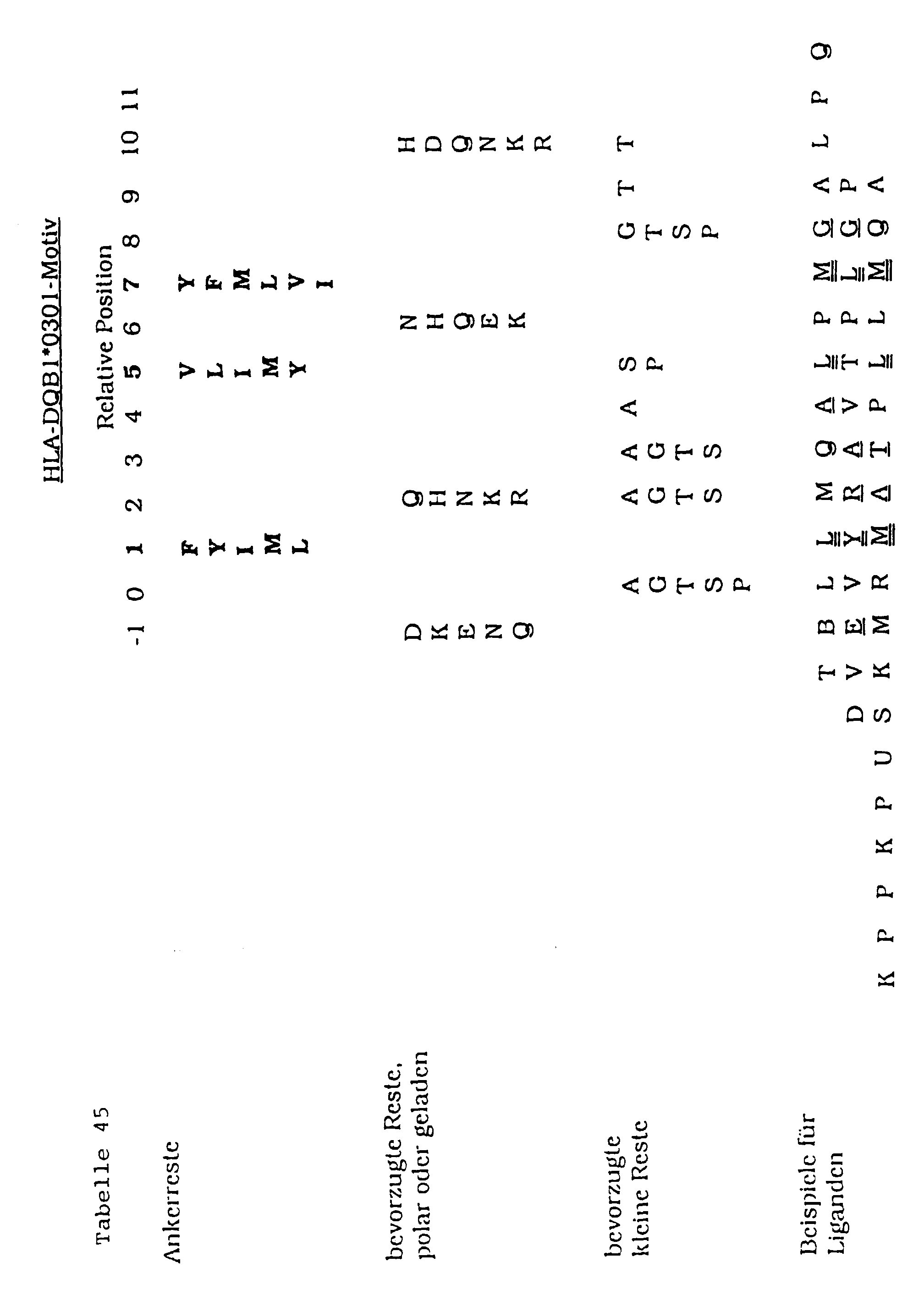

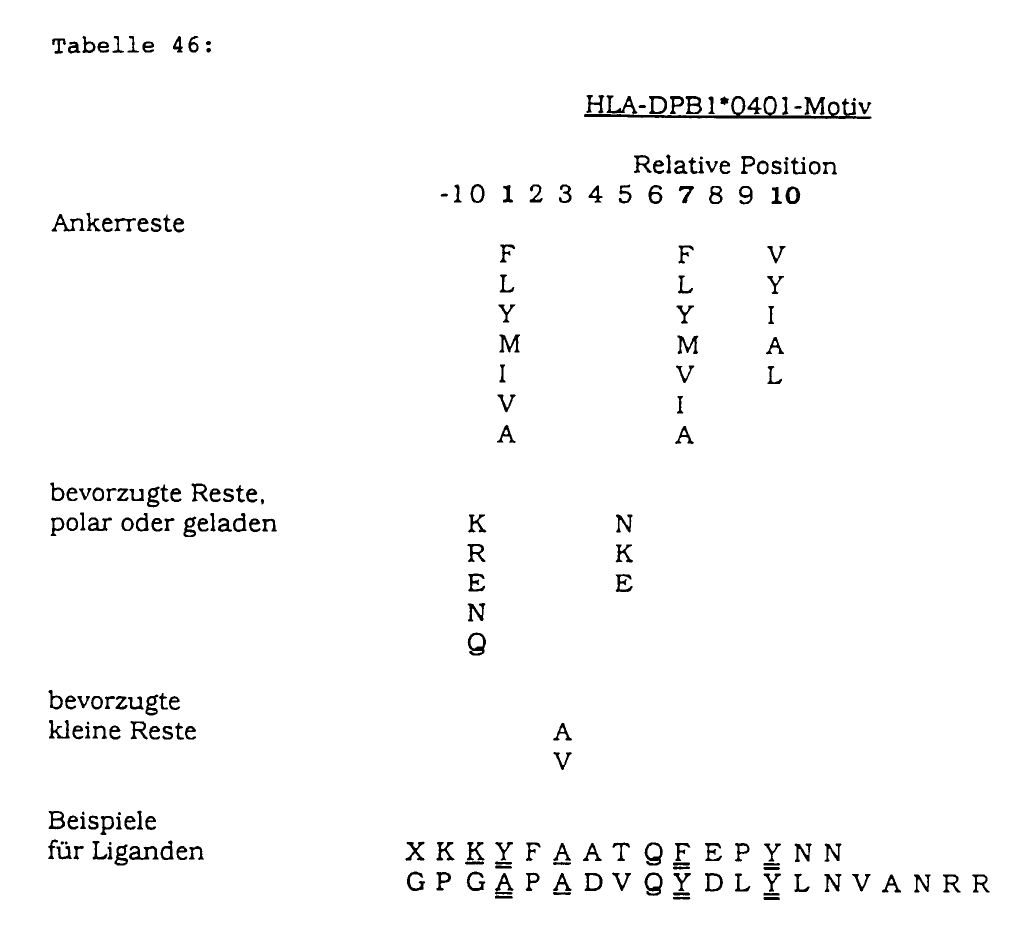

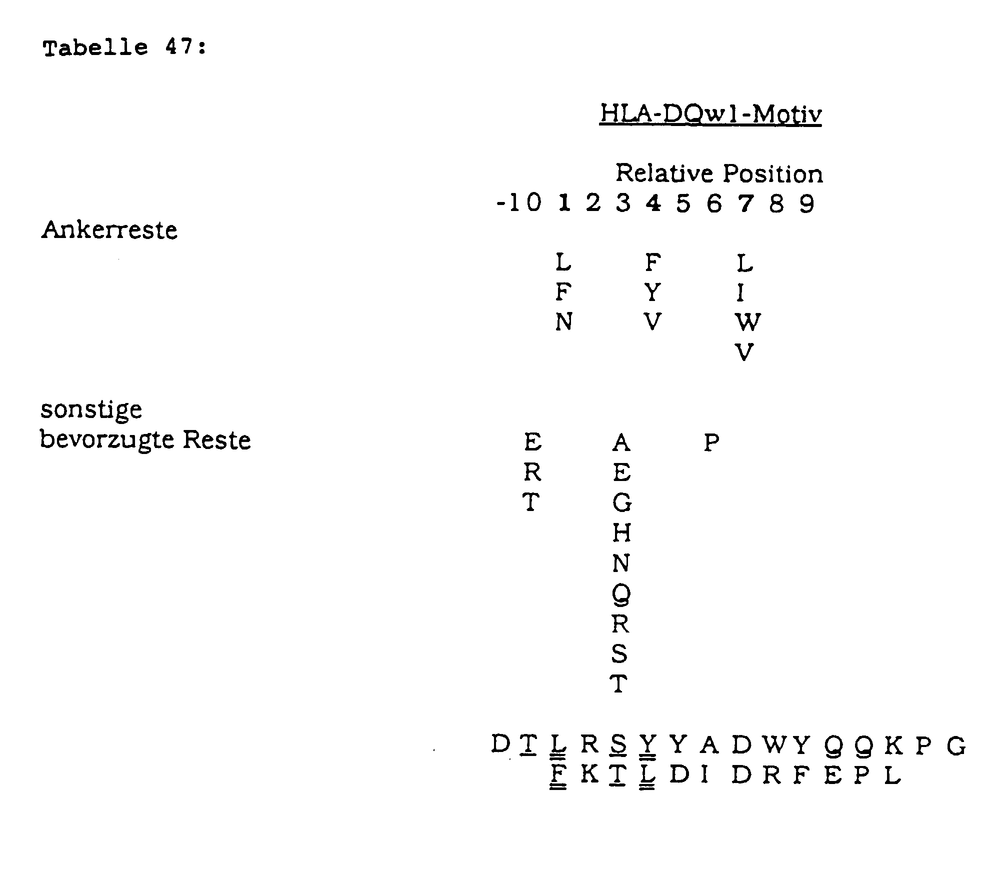

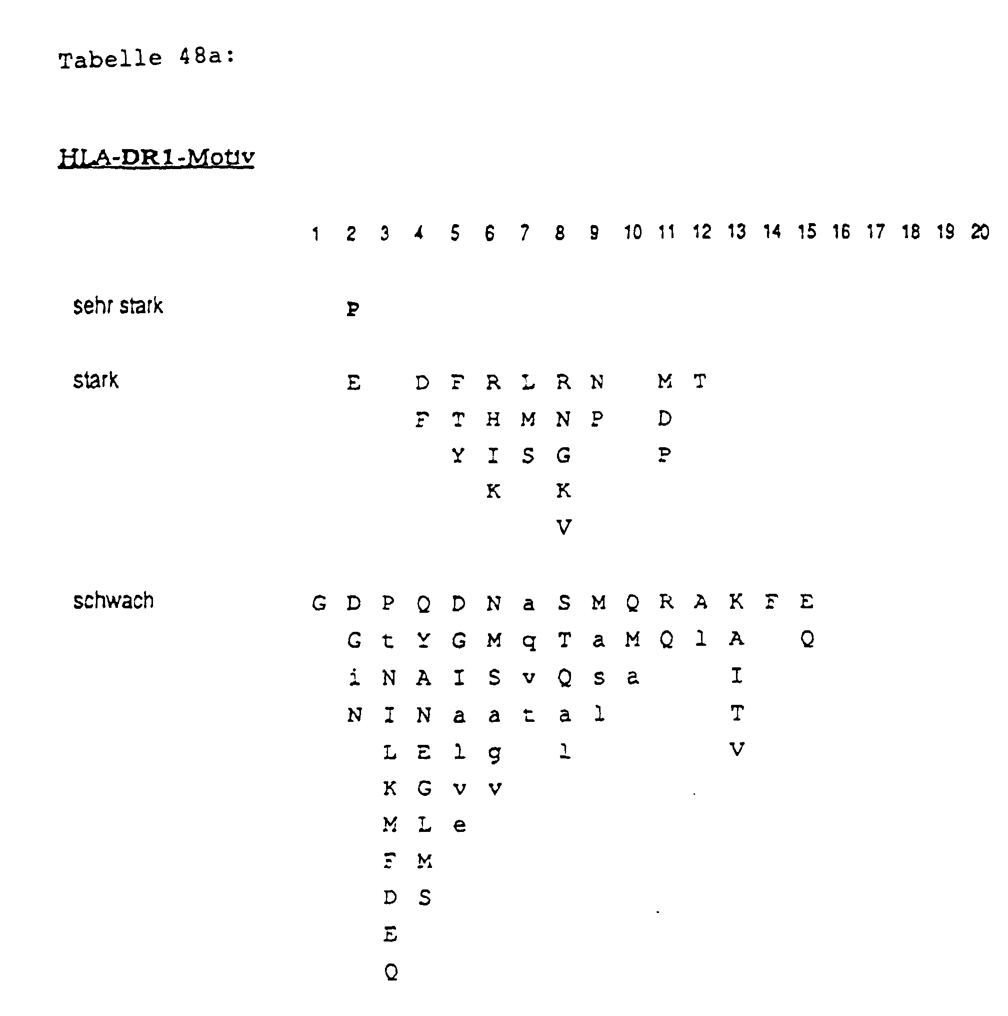

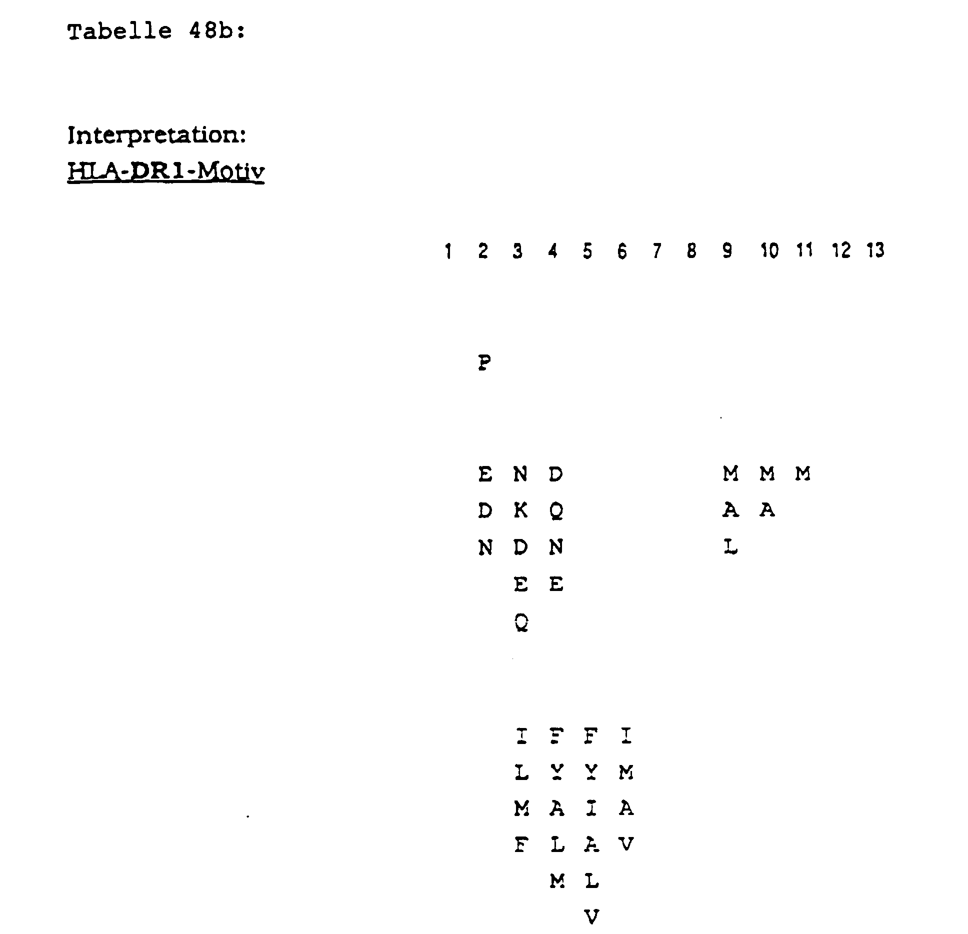

Demnach haben natürliche DR1-Liganden folgende Eigenschaften:
1.) Länge überwiegend mehr als 11 Aminosäuren (ist bereits bekannt)
2.) Überwiegend P an zweiter Position
3.) Polare/geladene Reste E, D, K, N, K, Q an Position 2, 3 oder 4
4.) Hydrophobe Reste I, L, M, F, A an Position 3, 4, 5 oder 6
5.) Hydrophobe Reste M. A oder L an Position 9. 10 oder 11

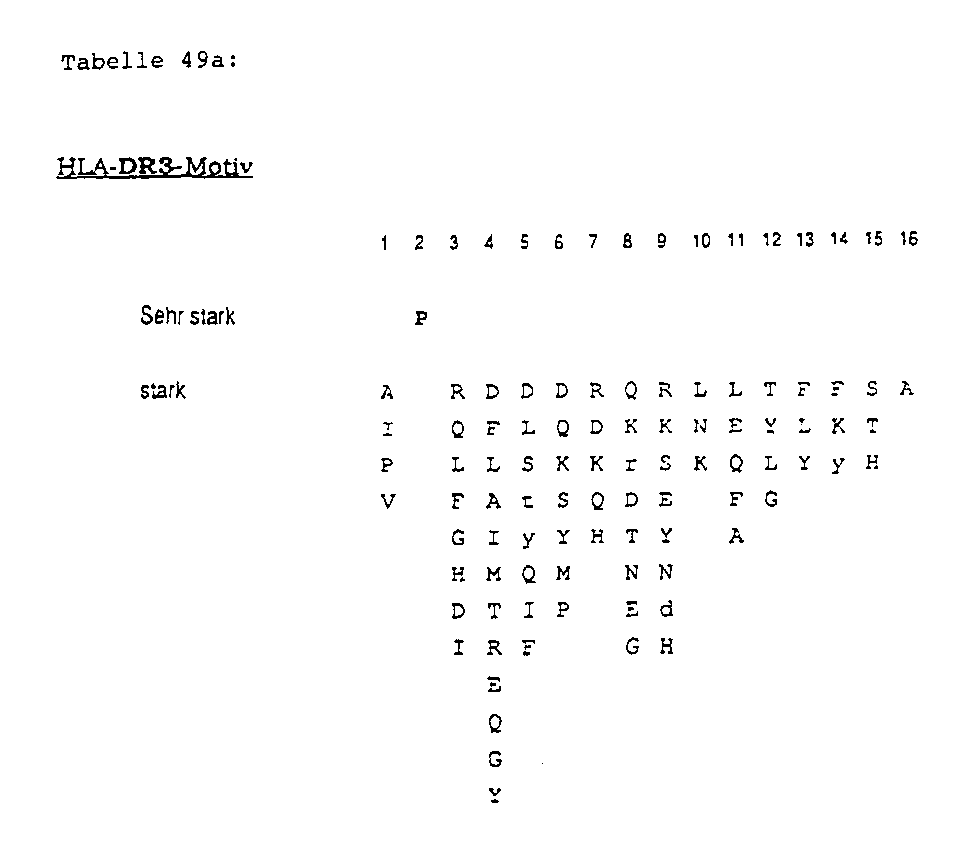

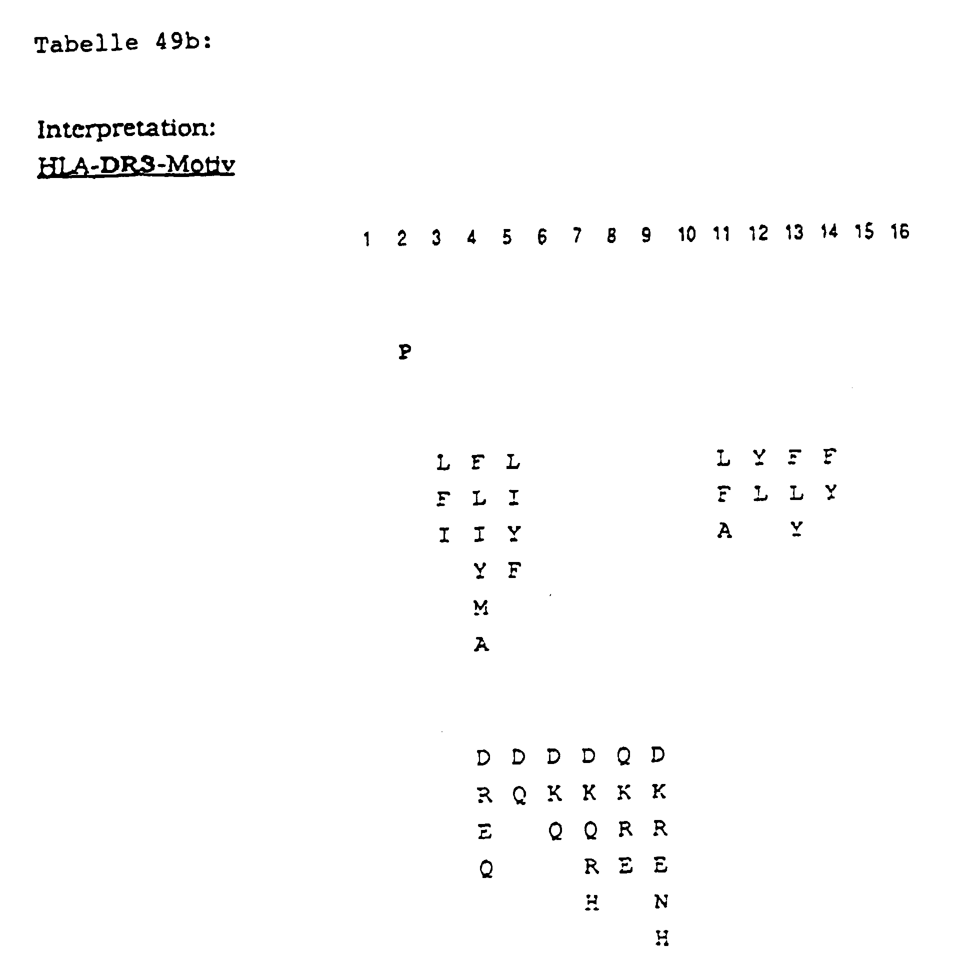

Demnach haben natürliche DR3-Liganden folgende Eigenschaften:
1.) wie bei DR1
2.) wie bei DR1
3.) Hydrophobe Reste L, F, l, Y, M, A an Position 3,4 oder 5
4.) Polare/geladene Reste an Position 4, 5, 6, 7, 8 oder 9
5.) Hydrophobe Reste L, F, A, Y an Position 11, 12 oder 13

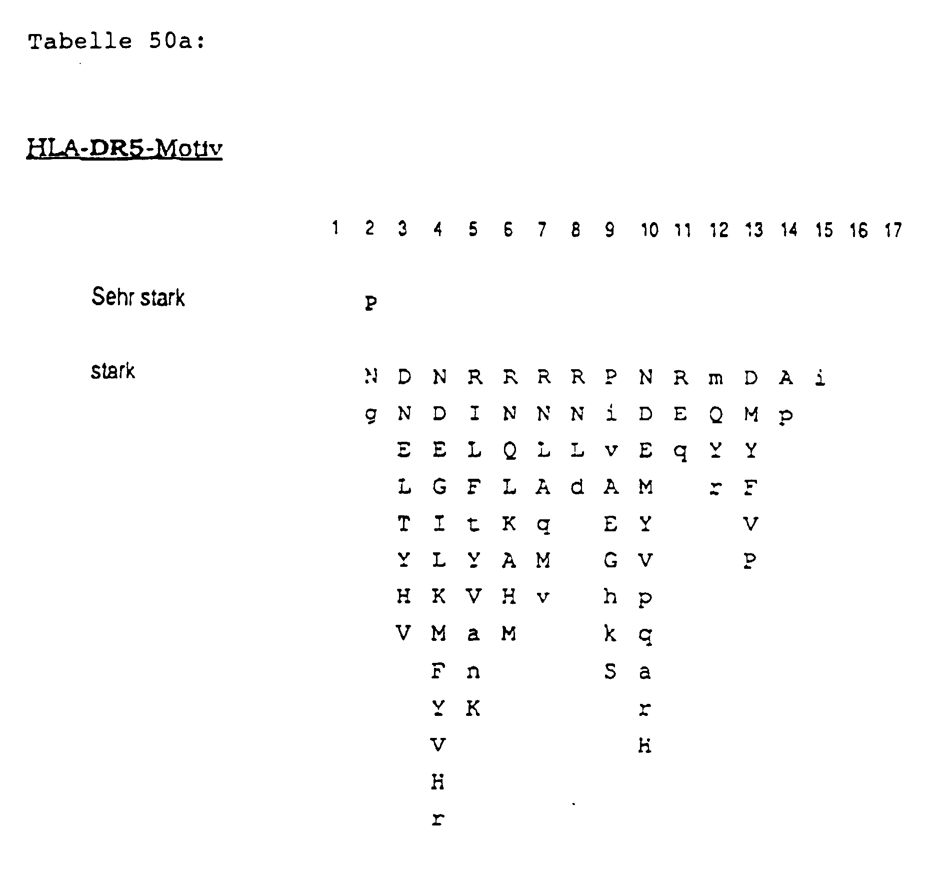

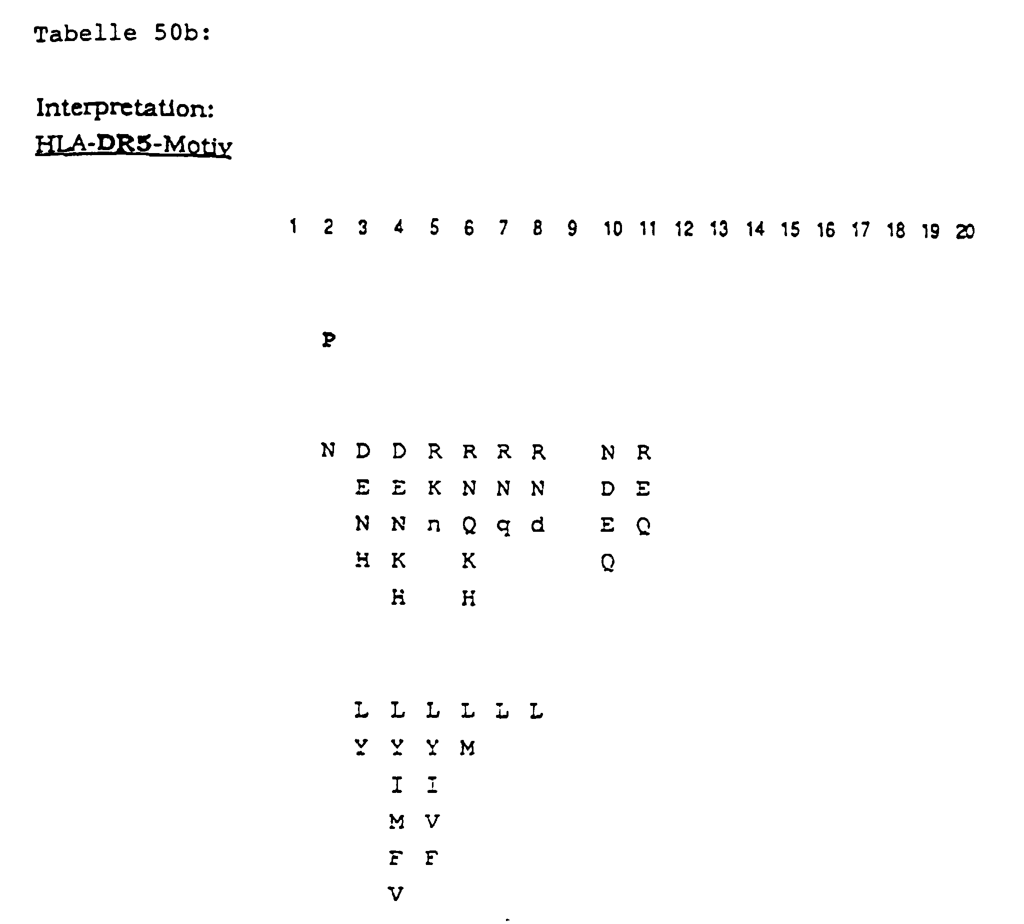

Demnach haben natürliche DR5-Liganden folgende Eigenschaften:
1.) wie DR1
2.) wie DR1
3.) Polare/überwiegend negativ geladene Reste N, D, E, H, K an Position 2, 3 oder 4
4.) Polare/überwiegend positiv geladene Reste R, K, N, Q an Position 5, 6, 7 oder 8
5.) Hydrophobe Reste L, Y, V, I, M, F, A an Position 3, 4 oder 5 sowie an 5, 6 oder 7
6.) Polare/geladene Reste N, D, E, H, R, Q an Position 10, 11 oder 12

### Literaturstellen

1. Zinkernagel, R.M. & Doherty, P.C., **Nature** 248, 701-702 (1974).
2. Townsend, A.R. et al., Cell 44, 959-968 (1986).
3. Bjorkman, P.J. et al., **Nature** 329, 512-518 (1987).
4. Rötzschke, O. et al., **Nature** 348, 252-254 (1990).
5. VanBleck, G.M. & Nathenson, S.G., **Nature** 348, 213-216 (1990).
6. Garrett, T.P.J., Saper, M.A., Bjorkman, P.J., Strominger, J.L. & Wiley, D.C., **Nature** 343, 692-696 (1989).
7. Rötzschke, O., Falk, K., Wallny, H.-J., Faath, S. & Rammensee, H.-G., Science 249, 283-287 (1990).
8. Falk, K., Rötzschke, O. & Rammensee, H.-G., Nature 348, 248-251 (1990).
9. Shimorkevitz, R., Kappler, J., Marrack, P. & Grey H., **J.exp.Med**. 158, 303-316 (1983).
10. Demotz, S., Grey, H.M., Appella, E. & Sette, A., **Nature** 343, 682-684 (1989).
11. Bjorkman, P.J. et al., **Nature** 329, 506-512 (1987).
12. DeLisi, C. & Berzolsky, J.A., Proc.natn.Acad.Sci.USA 82, 7048-7052 (1985).
13. Rothbard, J.B. & Taylor, W.R., **EMBO** J. 7, 93-100 (1988).
14. Cornette, J.L., Margaht, H., DeLisi, C. & Berzolsky, J.A., **Meth.Enzym** 178, 611-633 (1989).
15. Sette, A. et al., **Proc.natn.Acad.Sci.USA** 86, 3296-3300 (1989).
16. Maryanski, J.L., Verdini, A.S., Weber, P.C., Salemme, F.R. & Corradin, G., Cell 60, 63-72 (1990).
17. Bastin, J., Rothbard, J. Davey, J. Jones, I. & Townsend, A., **J.exp.Med**. 165, 1508-1523 (1987).
18. Bjorkman, P.J. & Davis, M.M., **Cold Spring Harb.Symp. quant.Biol.** 54, 365-374 (1989).
19. Boulliot, M. et al., **Nature** 339, 473-475 (1989).
20. Frelinger, J.A., Gotch, F.M., Zweerink, H., Wain, E. & McMichael, A.J., **J.exp.Med.** 172, 827-834 (1990).
21. Schild, H., Rötzschke, O., Kalbacher, H. & Rammensee, H.-G., **Science 247**, 1587-1589 (1990).
22. Townsend, A. et al., **Nature** 340, 443-448 (1989).
23. Elliott, T., Townsend, A. & Cerundolo, V., **Nature 348**, 195-197 (1990).
24. Cerundolo, V. et al., **Nature** 345, 449-452 (1990).
25. Rüsch, E., Kuon, W. & Hämmerling, G., **J.Trans.Proc**. 15, 2093-2096 (1983).
26. Lembke, H., Hämmerling, G.J. & Hämmerling U., **Immunol.Rev.** 47, 175-206 (1979).
27. Ozato, K. & Sachs, D.H., **J.Immun**. 126, 317-321 (1981).
28. Parham, P. & Brodsky, F.M., **Hum.Immun**. 3, 277-299 (1981).
29. Taylor, P.M., Davey, J., Howland, K., Rothbard, J.B. & Askonas, B.A., **Immunogenetics** 26, 267-272 (1987).
30. Braciale, T.J. et al., **J.exp.Med**. 166, 678-692 (1987).
31. Braciale, T.J., Sweetser, M.T., Morrison, L.A., Kittlesen, D.J. & Braciale, V.L., **Proc.natn.Acad.Sci.USA** 86, 277-281 (1989).
32. Kuwano, K., Braciale, T.J. & Ennis, F.A., **FASEB J**. 2, 2221 (1988).
33. Maryanski, J.L., Pala, P., Cerottini, J.C. & Corradin, G.J., **J.Exp.Med**. 167, 1391-1405 (1988).
34. Maryanski, J.L., Pala, P., Corradin, G., Jordan, B.R. & Cerottini, J.C., **Nature** 324, 578-579 (1986).
35. Sibille, C. et al., **J.exp.Med**. 172, 35-45 (1990).
36. Romero, P. et al., **Nature** 341, 323-326 (1989).
37. Weiss, W.R. et al., **J.exp.Med**. 171, 763-773 (1990).
38. Kast, W.M. et al., **Cell** 59, 603-614 (1989).
39. Oldstone, M.B.A., Whitton, J.L., Lewicki, H. & Tishon, A., **J.exp.Med**. 168, 559-570 (1988).
40. Tevethia, S.S. et al., **J.Virol**. 64, 1192-1200 (1990).
41. Carbone, F.R. & Bevan, M.J., **J.exp.Med**. 169, 603-612 (1989).
42. Schumacher, T.N.M. et al., Cell 62, 563-567 (1990).
43. Walker, B.D. et al., **Proc.natn.Acad.Sci.USA** 86, 9514-9518 (1989).
44. Gotch, F., McMichael, A. & Rothbard, J., **J.exp.Med**. 168, 2045-2057 (1988).
45. Santos-Aguado, J., Commins, M.A.V., Mentzer, S.J., Burakoff, S.J. & Strominger, J.L., **Proc.natn.Acad.Sci.USA** 86, 8936-8940 (1989).
46. Clavene, J.M. et al., **Eur.J.Immun**. 18, 1547-1553 (1988).
47. Falk, K. et al., **J.exp.Med**. A4, 425-434 (1991).

## Patentansprüche

1. Verwendung von allelspezifischen Peptidmotiven auf Molekülen des Major Histocompatibility Complex (MHC) der Klassen I oder II, die aus der Gruppe, bestehend aus HLA-A1, HLA-A3, HLA-A11, HLA-A24, HLA-A31, HLA-A33, HLA-B7, HLA-B8, HLA-B*2702, HLA-B*3501, HLA-B*3503, HLA-B37, HLA-B38, HLA-B*3901, HLA-B∗3902, HLA-B*5101, HLA-B*5102, HLA-B*5103, HLA-B*5201, HLA-B58, HLA-B60, HLA-B61, HLA-B62, HLA-B78, HLA-Cw*0301, HLA-Cw*0401, HLA-Cw*0602, HLA-Cw*0702, HLA-Cw4, HLA-Cw6, HLA-Cw7, HLA-DRB1*0101, DRB1*1201, HLA-DR4w14, HLA-DR17, HLA-DRw52, HLA-DPw2, HLA-DPB1*0401, HLA-DQB1*0301 und HLA-DQw1 ausgewählt sind, und dadurch erhältlich sind, daß man
(a) durch Zellaufschluß von Zellen, die entsprechende MHC-Moleküle enthalten, einen Zellextrakt erzeugt,
(b) MHC-Moleküle mit den darauf befindlichen Peptidmischungen durch Immunpräzipitation aus dem Zellextrakt abtrennt,
(c) die Peptidmischungen von MHC-Molekülen und sonstigen Proteinbestandteilen abtrennt,
(d) einzelnen Peptide oder/und ein Gemisch davon sequenziert, und
(e) aus den erhaltenen Informationen, insbesondere aus der Sequenzierung eines Gemisches, oder aus der Sequenzierung einer Reihe von Einzelpeptiden, das jeweilige allelspezifische Peptidmotiv gemäß einer der Tabellen 8-47 ableitet,
bei einem Verfahren zur Herstellung eines diagnostischen oder therapeutischen Mittels.

2. Verwendung nach Anspruch 1 zur Herstellung eines Mittels für den diagnostischen Nachweis von MHC-Molekülen.

3. Verwendung nach Anspruch 1 oder 2,
**dadurch gekennzeichnet,** daß man ein Peptid, das einem Peptidmotiv entspricht, mit einer Markierungsgruppe, insbesondere einer Biotin- oder einer Fluoreszenzgruppe koppelt.

4. Verwendung nach Anspruch 1 zur Herstellung eines Mittels für die Therapie von Störungen des Immunsystems oder von Tumorerkrankungen.

5. Verwendung nach Anspruch 4 für die Therapie von Autoimmunkrankheiten, Transplantatabstoßungen oder/und Graft-versus-Host-Reaktionen.

6. Verwendung nach einem der Ansprüche 1 bis 5,
**dadurch gekennzeichnet,** daß ein Peptid, das einem Peptidmotiv entspricht, N- oder/und C-terminal mit lipophilen bzw. amphiphilen Gruppen, insbesondere auch lipophilen Peptid-Helices kovalent verknüpft wird.

7. Verwendung nach Anspruch 6,
**dadurch gekennzeichnet,** daß die lipophile bzw. amphiphile Gruppe Tripalmitoyl-S-glycerylcysteinyl-serylserin ist.

## Claims

1. Use of allele-specific peptide units on molecules of the class I or class II major histocompatibility complex (MHC) which are selected from the group consisting of HLA-A1, HLA-A3, HLA-A11, HLA-A24, HLA-A31, HLA-A33, HLA-B7, HLA-B8, HLA-B*2702, HLA-B*3501, HLA-B*3503, HLA-B37, HLA-B38, HLA-B*3901, HLA-B*3902, HLA-B*5101, HLA-B*5102, HLA-B*5103, HLA-B*5201, HLA-B58, HLA-B60, HLA-B61, HLA-B62, HLA-B78, HLA-Cw*0301, HLA-Cw*0401, HLA-Cw*0602, HLA-Cw*0702, HLA-Cw4, HLA-Cw6, HLA-Cw7, HLA-DRB1*0101, DRB1*1201, HLA-DR4w14, HLA-DR17, HLA-DRw52, HLA-DPw2, HLA-DPB1*0401, HLA-DQB1*0301 and HLA-DQw1, and which are obtainable by a procedure wherein
(a) a cell extract is produced by digesting cells which contain appropriate MHC molecules,
(b) MHC molecules with the peptide mixtures present thereon are separated from the cell extract by immunoprecipitation,
(c) the peptide mixtures are separated from MHC molecules and other protein constituents,
(d) individual peptides and/or a mixture thereof are sequenced, and
(e) the respective allele-specific peptide unit is derived from the information obtained, especially from the sequencing of a mixture or from the sequencing of a number of individual peptides, according to one of Tables 8 - 47,
in a process for the preparation of a diagnostic or therapeutic agent.

2. Use according to Claim 1 for the preparation of an agent for the diagnostic detection of MHC molecules.

3. Use according to Claim 1 or 2, characterized in that a peptide corresponding to a peptide unit is coupled with a labelling group, especially a biotin group or a fluorescent group.

4. Use according to Claim 1 for the preparation of an agent for the therapy of immune system disorders or tumoural diseases.

5. Use according to Claim 4 for the therapy of autoimmune diseases, graft rejections and/or graft-versus-host reactions.

6. Use according to one of Claims 1 to 5, characterized in that a peptide corresponding to a peptide unit is covalently linked to lipophilic or amphiphilic groups, especially lipophilic peptide helices, at the N-terminus and/or C-terminus.

7. Use according to Claim 6, characterized in that the lipophilic or amphiphilic group is tripalmitoyl-S-glycerylcysteinylserylserine.

## Revendications

1. Utilisation de motifs peptidiques spécifiques d'allèles sur des molécules du complexe majeur d'histocompatibilité (CMH) de classe I ou II, qui sont choisis dans le groupe consistant en HLA-A1, HLA-A3, HLA-A11, HLA-A24, HLA-A31, HLA-A33, HLA-B7, HLA-B8, HLA-B*2702, HLA-B*3501, HLA-B*3503, HLA-B37, HLA-B38, HLA-B*3901, HLAB*3902, HLA-B*5101, HLA-B*5102, HLA-B*5103, HLA-B*5201, HLA-B58, HLA-B60, HLA-B61, HLA-B62, HLA-B78, HLA-Cw*0301, HLA-Cw*0401, HLA-Cw*0602, HLA-Cw*0702, HLA-Cw4, HLA-Cw6, HLA-Cw7, HLA-DRB1*0101, DRB1*1201, HLA-DR4w14, HLA-DR17, HLA-DRw52, HLA-DPw2, HLA-DPB1*0401, HLA-DQB1*0301 et HLA-DQw1, et qui peuvent être obtenus par le fait que
(a) on produit un extrait cellulaire par lyse de cellules qui contiennent des molécules du CMH correspondantes,
(b) on sépare de l'extrait cellulaire, par immunoprécipitation, des molécules du CMH avec les mélanges de peptides présents sur celles-ci,
(c) on sépare les mélanges de peptides des molécules du CMH et des autres constituants protéiques,
(d) on séquence des peptides individuels et/ou un mélange de ceux-ci, et
(e) d'après les informations obtenues, en particulier d'après le séquençage d'un mélange ou d'après le séquençage d'une série de peptides individuels, on déduit le motif peptidique spécifique d'allèles correspondant selon l'un des tableaux 8-47,
dans un procédé de préparation d'un agent diagnostique ou thérapeutique.

2. Utilisation selon la revendication 1 pour la préparation d'un agent pour la mise en évidence diagnostique de molécules du CMH.

3. Utilisation selon la revendication 1 ou 2 caractérisée en ce que l'on couple un peptide qui correspond à un motif peptidique avec un groupe de marquage, en particulier un groupe biotine ou un groupe fluorescent.

4. Utilisation selon la revendication 1 pour la préparation d'un agent pour la thérapie de troubles du système immunitaire ou de maladies tumorales.

5. Utilisation selon la revendication 4 pour la thérapie de maladies auto-immunes, de rejets de greffes et/ou de réactions du greffon contre l'hôte.

6. Utilisation selon l'une des revendications 1 à 5 caractérisée en ce qu'un peptide qui correspond à un motif peptidique est lié de manière covalente à l'extrémité N-et/ou C-terminale avec des groupes lipophiles ou amphiphiles, en particulier aussi avec des hélices peptidiques lipophiles.

7. Utilisation selon la revendication 6 caractérisée en ce que le groupe lipophile ou amphiphile est la tripalmitoyl-S-glycérylcystéinyl-sérylsérine.
